(19) Europäisches Patentamt
European Patent Office
Office européen des brevets

(11) EP 0 441 875 B1

(12) EUROPEAN PATENT SPECIFICATION

(45) Date of publication and mention of the grant of the patent:
**09.07.1997 Bulletin 1997/28**

(21) Application number: **89912852.4**

(22) Date of filing: **03.11.1989**

(51) Int Cl.6: **G01N 33/52**, G01N 33/536, G01N 33/58

(86) International application number:
**PCT/US89/04915**

(87) International publication number:
**WO 90/05302 (17.05.1990 Gazette 1990/11)**

(54) **ENHANCED ELECTROCHEMILUMINESCENCE**
GESTEIGERTE ELEKTROCHEMILUMINESZENZ
ELECTROCHIMIOLUMINESCENCE AMELIOREE

(84) Designated Contracting States:
**AT BE CH DE FR GB IT LI LU NL SE**

(30) Priority: **03.11.1988 US 267509**

(43) Date of publication of application:
**21.08.1991 Bulletin 1991/34**

(73) Proprietor: **IGEN, INC.**
**Gaithersburg, Maryland 20877 (US)**

(72) Inventors:
- **SHAH, Haresh, P.**
  **T-4, Gaitherburg, MD 20878 (US)**
- **VON BORSTEL, Reid, W.**
  **Kensington, MD 20895 (US)**
- **TYAGI,Surendera,K.**
  **Mundelein,Illinois 60060, (US)**

(74) Representative: **Cropp, John Anthony David et al**
**MATHYS & SQUIRE**
**100 Grays Inn Road**
**London, WC1X 8AL (GB)**

(56) References cited:

| | |
|---|---|
| **EP-A- 0 096 749** | **WO-A-86/02734** |
| **WO-A-87/06707** | **US-A- 4 372 745** |

- **See also references of WO9005302**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 441 875 B1

## Description

### Field of the Invention

This application relates generally to electrochemiluminescent ("ECL") reactions, and more particularly to detecting the presence of an analyte of interest, and, if desired, quantitating the amount present, by measurement of electromagnetic radiation emitted by the system investigated.

The reader is referred to WO 87/06706, EP-A-0 339 086, EP-A-0 417 140 and EP-A-0 446 245.

### Background of the Invention

Early ECL reactions involved the annihilation of oppositely charged radical ions, produced by sequential oxidation and reduction at an electrode using a double potential step. See Faulkner, L. R. et al., Electroanalytical Chemistry, A. J. Bard (Ed.), Vol. 10, Marcel Dekker, N. Y., 1977, Ch. 1; Tokel-Takvoryan, N. E., et al., Chem. Phys. Lett., 1974, 25, 235; Velasco, J. C., et al., Inorg. Chem. 1983, 22, 822; Luong, J. C., et al., J. Am. Chem. Soc. 1978, 100, 5790; Abruna, H. D., J. Electrochem. Soc. 1985, 132, 842; and Abruna, H. D., J. Electroanal. Chem. 1984, 175, 321. Upon homogeneous electron transfer between the sufficiently energetic and oppositely charged radicals, an excited state of one of the precursors can be formed, and subsequent emission by the species in the excited state occurs. Additionally, so-called energy deficient mechanisms involving triplet-triplet annihilations have been reported. See Freed, D. et al., J. Am. Chem. Soc. 1971, 93, 2097; Wallace, W. L. et al., J. Electrochem. Soc. 1978, 125, 1430.

In certain other ECL reactions luminophore has been used with a carboxylic acid, such as oxalate or pyruvate, to achieve electrochemiluminescence. Oxidative-reduction mechanisms such as this involve the oxidation of $Ru(bpy)_3 2+$ (herein, "bpy" stands for "bipyridyl"), and the carboxylic acid. See Ege, D., et al., J. Anal. Chem. 1984, 56, 2413; Rubinstein, I., et al., J. Am. Chem. Soc. 1981, 103, 512; Chan, M.M., et al., J. Am. Chem. Soc. 1979, 99, 5399. However, the amount of luminophore reportedly needed for these systems is undesirably large in many cases. It would be a desirable advance for these systems to be effective when using a smaller amount of luminophore than is ordinarily the case for detecting an analyte sample of given size. It would also be desirable for these systems to have an enhanced detection capability, permitting sensing of a smaller amount of analyte than is ordinarily the case for a given amount of luminophore.

Accordingly, provision of materials and methods for conducting ECL reactions exhibiting a favorable detection limit would be a significant technological advance.

The present invention provides a means of enhancing the level of emitted electromagnetic radiation.

According to the present invention, there is provided the use of a compound or compounds of the formula

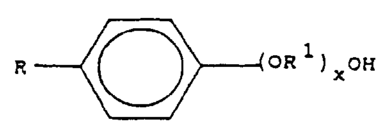

where R is hydrogen or $C_nH_{2n+1}$,$R^1$ is $C_nH_{2n}$ x is 0 to 70 and n is from 1 to 20, as an electromagnetic radiation enhancer in a composition suitable for use in an ECL assay and comprising

(a) a metal-containing ECL moiety which, when oxidized by exposure to an effective amount of electrochemical energy, is capable of being converted to an excited state from which electromagnetic radiation is emitted upon exposure of the excited ECL moiety to conditions sufficient to induce said emission;
(b) a species, which, when oxidized, forms a strong reducing agent;
(c) an electrolyte capable of functioning as a medium in which said ECL moiety and said species can be oxidized.

In accordance with one aspect of the invention, the composition is in the form of a kit comprising at least one separate component including one or more members of the group consisting of components (a), (b) and (c) and said electromagnetic radiation enhancing compound. The kit may comprise a first separate component including any two members of the said group and a second separate component including the remaining members of the group. Said second separate component may additionally include either one of the other members of the group in the first component. In another embodiment, the kit may comprise four separate compounds each of which includes a different one of the four members of the group.

In yet another embodiment the kit comprises a first separate component including all four members of the group and a second separate component including any one, two or three members of the group. In one aspect of this embodiment the second separate component includes one of the four members of the group and the kit further comprises a third separate component including one, two or three members of the group and a fourth separate component including one or two members of the group.

In still a further embodiment, the kit comprises a first separate component including at least two members of the group and a second separate component including the remaining member or members.

The invention further provides the use of a compound or compounds of the formula

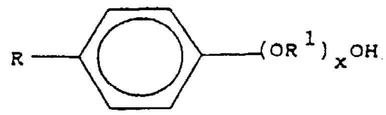

where R is hydrogen or $C_nH_{2n+1}$, $R^1$ is $C_nH_{2n}$, x is 0 to 70 and n is from 1 to 20, as an electromagnetic radiation enhancer in a method of generating emission of electromagnetic radiation which comprises the steps of

(i) forming a composition as defined above;
(ii) exposing the composition under suitable conditions to an amount of electrochemical energy effective to induce the composition to emit electromagnetic energy; and
(iii) detecting the emitted electromagnetic radiation;

said compound being included in said composition. The method may be a method of detecting or quantitating an analyte of interest by ECL assay wherein the composition further includes

(A) a sample to be tested for the analyte of interest,
(B) at least one substance selected from the group consisting of

(i) additional analyte of interest or an analog of the analyte of interest,
(ii) a binding partner of the analyte of interest or its said analog,
and
(iii) a reactive component capable of binding with (i) or (ii), and wherein the metal-containing ECL moiety is a metal chelate which is capable of entering into a binding interaction with the analyte of interest or with (B)(i), (B)(ii), or (B)(iii).

According to the invention, there is also provided the use of a compound or compounds of the formula

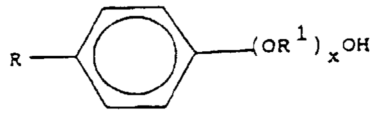

where R is hydrogen or $C_nH_{2n+1}$, $R^1$ is $C_nH_{2n}$, x is 0 to 70 and n is from 1 to 20, as an electromagnetic radiation enhancer in a system for detecting or quantitating an analyte of interest in a sample based upon an electrochemiluminescent phenomenon comprising:

(A) a sample;
(B) at least one substance selected from the group consisting of

(i) added analyte of interest or an analog of the analyte of interest,
(ii) a binding partner of the analyte of interest or its said analog, and
(iii) a reactive component capable of binding with (i) or (ii), wherein one of said substances is linked directly or by one or more other molecules to a metal-containing ECL moiety which is capable of being converted to an excited state from which electromagnetic radiation is emitted upon exposure of the ECL moiety to conditions sufficient to induce said emission;
(C) a species which is capable of being converted to a strong reducing agent and an electrolyte;
(D) means for inducing the ECL moiety to emit electromagnetic radiation; and
(E) means for measuring the radiation emitted by said composition to determine the presence or quantity of

the analyte of interest in the sample.

The "ECL moiety" or "metal-containing ECL moiety" is sometimes referred to as a "label," "label compound," "label substance," etc. It is within the scope of the invention for the "ECL moiety," "metal-containing ECL moiety," "organometallic," "metal chelate," "transition metal chelate" and "rare earth metal chelate" -- when utilized in certain of the composition, reagent, kit, method or system embodiments in accordance with the inventions-- to be linked to other molecules such as an analyte or an analog thereof, binding partner of such analyte or an analog thereof, further binding partner of the aforementioned binding partner, reactive component capable of binding with the analyte, an analog thereof or a binding partner as mentioned above. The above-mentioned species can also be linked to a combination of one or more binding partners and/or one or more reactive components. Additionally, the aforementioned species can also be linked to an analyte or its analog bound to a binding partner, a reactive component, or a combination of one or more binding partners and/or one or more reactive components. It is also within the scope of the invention for a plurality of the aforementioned species to be bound directly, or through other molecules as discussed above, to an analyte or its analog.

It is similarly within the scope of the invention for the aforementioned "composition" (hereinafter sometimes an "ECL composition") or "system" to contain unstable, metastable and other intermediate species formed in the course of the ECL reaction, such as an ECL moiety in an excited state as aforesaid and the above-mentioned strong reducing agent.

Additionally, although the emission of visible light is an advantageous feature of certain embodiments of the invention it is within the scope of the invention for the composition (or ECL composition) to emit other types of electromagnetic radiation, such as infrared or ultraviolet light, X-rays, microwaves, etc. Use of the terms "electrochemiluminescence," "electrochemiluminescent," "electrochemiluminesce," "luminescence," "luminescent" and "luminesce" in connection with the present invention does not require that the emission be light, but admits of the emission's being such other forms of electromagnetic radiation.

Substantial advantages are conferred on the practitioner of the present invention. The materials and methods in accordance with the invention provide an elegant technology for conducting the ECL detection and quantitation of an analyte of interest over a wide concentration range, down to a very small analyte concentration, in aqueous as well as organic environments. Good precision, and repeatability of detection and quantitation measurements, are obtained. Smaller concentrations of analyte than would otherwise be detectable with conventional systems can be sensed. Conversely, other things being equivalent, less metal-containing ECL moiety is required to detect an analyte sample of particular (especially small) concentration with the present invention than would be required with prior technology. Thus, an improved detection limit is realized with the invention.

Furthermore, the present invention is useful in the detection and quantitation of numerous and highly varied analytes of interest as is discussed in the further description of the invention which follows.

Additionally, the versatility of the present invention is further evident from the fact that it is not only useful in conducting heterogeneous assays, but also homogeneous assays. In this connection, heterogeneous assays are those in which ECL moiety linked directly or through one or more other molecules to the analyte of interest or its analog is separated, prior to exposure of such ECL moiety to electrochemical energy, from ECL moiety not linked to the analyte or its analog. Homogeneous assays, by way of contrast, are those in which there is no such separation before exposing the materials to electrochemical energy together. In the homogeneous assays of the present invention, electromagnetic radiation emitted when the ECL moiety is linked to the analyte or its analog differs from electromagnetic radiation emitted when the ECL moiety is not linked to the analyte or its analog. This can be achieved, for example, by sensing an increased or decreased emission corresponding to the presence of ECL moiety linked to analyte or its analog.

## Brief Description of the Drawings

Figure 1 is a schematic drawing of a cell suitable for inducing the emission of electrochemiluminescence in accordance with the present invention.

Figure 2 is a simplified diagram of a voltage control apparatus for use with the cell illustrated in Figure 1.

Figure 3 is a plot of ECL intensity (Counts) versus the concentration (nM) of TAG (tris (2,2'-bipyridyl) ruthenium chloride hexahydrate).

Figure 4 graphically depicts the results of a homogeneous ECL theophylline assay.

Figure 5 graphically depicts the results of a homogeneous theophylline assay in various sera.

• = Normal sera
■ = Hemolyzed sera
⧫ = Lipemic sera
= Icteric sera

Figure 6 graphically depicts the results of an ECL theophylline assay compared to the results of a fluorescence polarization theophylline assay.

A. Normal sera: n = 4; slope = .986; r = 1.00.
B. Hemolyzed sera: n = 3; slope = .878; r = 1.00
C. Lipemic sera: n = 5; slope = .872; r = 0.99
D. Icteric sera: n = 4; slope = 2.14; r = 1.00

Figure 7 graphically depicts the results of an ECL digoxin immunoassay.

■ = Blank
• = Digoxin

Description of Certain Preferred Embodiments

The invention, as well as additional objects, features and advantages thereof, will be understood more fully from the following detailed description of certain preferred embodiments.

The invention is useful in enabling the detection and quantitation of metal-containing compounds such as metal chelates and of other analytes of interest which are capable of entering into a binding reaction. These reactions include, for example, antigen-antibody reactions, ligand receptor reactions, DNA and RNA interactions, and other known reactions. In certain embodiments the invention relates to different materials and methods for qualitatively and quantitatively detecting the presence of analytes of interest in a multicomponent sample.

In addition to the metal-containing ECL moieties, typical analytes of interest are a whole cell or surface antigen, subcellular particle, virus, prion, viroid, antibody, antigen, hapten, nucleic acid, protein, lipoprotein, polysaccharide, lipopolysaccharide, glycoprotein, peptide, polypeptide, cellular metabolite, hormone, pharmacological agent, nonbiological polymer (preferably soluble), synthetic organic molecule, organometallic molecule, tranquilizer, barbituate, alkaloid, steroid, vitamin, amino acid, sugar, lectin, recombinant or derived protein, biotin, avidin, streptavidin, or inorganic molecule present in the sample. In one embodiment, the ECL moiety is conjugated to an antibody, antigen, nucleic acid, hapten, small nucleotide sequence, oligomer, ligand, enzyme, or biotin, avidin, streptavidin, Protein A, Protein G, or complexes thereof, or other secondary binding partner capable of binding to a primary binding partner through protein interactions.

Whole cells may be animal, plant, or bacterial, and may be viable or dead. Examples include plant pathogens such as fungi and nematodes. The term "subcellular particles" is meant to encompass, for example, subcellular organelles, membrane particles as from disrupted cells, fragments of cell walls, ribosomes, multienzyme complexes, and other particles which can be derived from living organisms. Nucleic acids include, for example, chromosomal DNA, plasmid DNA, viral DNA, and recombinant DNA derived from multiple sources. Nucleic acids also include RNA's, for example messenger RNA's, ribosomal RNA's and transfer RNA's. Polypeptides include, for example, enzymes, transport proteins, receptor proteins, and structural proteins such as viral coat proteins. Preferred polypeptides are enzymes and antibodies. Particularly preferred polypeptides are monoclonal antibodies. Hormones include, for example, insulin and T4 thyroid hormone. Pharmacological agents include, for example, cardiac glycosides. It is of course within the scope of this invention to include synthetic substances which chemically resemble biological materials, such as synthetic polypeptides, synthetic nucleic acids, and synthetic membranes, vesicles and liposomes. The foregoing is not intended to be a comprehensive list of the biological substances suitable for use in this invention, but is meant only to illustrate the wide scope of the invention.

Also, typically, the analyte of interest is present at a concentration of $10^{-3}$ molar or less, for example, at least as low as $10^{-18}$ molar.

The sample which may contain the analyte of interest, can be in solid, emulsion, suspension, liquid, or gas form, and can be derived from, for example, cells and cell-derived products, water, food, blood, serum, hair, sweat, urine, feces, tissue, saliva, oils, organic solvents or air. The sample can further comprise, for example, water, acetonitrile, dimethyl sulfoxide, dimethyl formamide, n-methylpyrrolidone or alcohols.

An essential feature of the invention is the utilization of metal-containing ECL moieties. Preferably, the ECL moiety is regenerative, so that it can repeatedly be induced to emit electromagnetic radiation, that is, it undergoes multiple emission events per molecule. This is a distinct advantage over conventional embodiments in which there is no "label" producing more than one emission event per molecule. (Note that it is within the scope of the invention to utilize additional labels such as radioactive isotopes, chemiluminescent molecules like luminol, etc.)

The ECL moieties utilized in accordance with the invention encompass organometallic compounds which emit electromagnetic radiation, such as visible light, as a result of electrochemical stimulation in accordance with the invention. Examples are 4,4',5',5 tetramethyl bipyridine Re(I)(4-ethyl-pyridine) $(CO_3)^+CF_3SO_3^-$; and Pt(2-(2-thienyl)pyrid-

ine)$_2$.

Advantageously, the metal-containing ECL moiety is a metal chelate. The metal of that chelate is such that the chelate emits electromagnetic radiation, such as visible light, as a result of electrochemical stimulation in accordance with the invention. The metal of such metal chelates is, for instance, a transition metal (such as a transition metal from the d-block of the periodic table) or a rare earth metal. The metal is preferably ruthenium, osmium, rhenium, iridium, rhodium, platinum, indium, palladium, molybdenum, technetium, copper, chromium or tungsten, or lanthanum, neodymium, praeseodymium or samarium. Especially preferred metals are ruthenium and osmium.

The ligands which are linked to the metal in such chelates are usually heterocyclic or organic in nature, and play a role in determining the emission wavelength of the metal chelate as well as whether or not the metal chelate is soluble in an aqueous environment or in an organic or other nonaqueous environment. The ligands can be polydentate and can be substituted. Suitable polydentate ligands include aromatic and aliphatic ligands. Such aromatic polydentate ligands include aromatic heterocyclic ligands. Preferred aromatic heterocyclic ligands are nitrogen-containing, such as, for example, bipyridyl, bipyrazyl, terpyridyl, and phenanthrolyl. Suitable substituents include for example, alkyl, substituted alkyl, aryl, substituted aryl, aralkyl, substituted aralkyl, carboxylate, carboxaldehyde, carboxamide, cyano, amino, hydroxy, imino, hydroxycarbonyl, aminocarbonyl, amidine, guanidinium, ureide, sulfur-containing groups, phosphorus containing groups, and the carboxylate ester of N-hydroxysuccinimide. The chelate can have one or more monodentate ligands, a wide variety of which are known to the art. Suitable monodentate ligands include, for example, carbon monoxide, cyanides, isocyanides, halides, and aliphatic, aromatic and heterocyclic phosphines, amines, stilbenes, and arsines.

Examples of suitable chelates are bis [(4,4'-carbomethoxy)-2,2'-bipyridine] 2-[3-(4-methyl-2,2'-bipyridine-4-yl)propyl]-1,3-dioxolane ruthenium (II); bis (2,2'bipyridine) [4-(butan-1-al)-4'-methyl-2,2'-bipyridine] ruthenium (II); bis (2,2'-bipyridine) [4-(4'-methyl-2,2'-bipyridine-4'-yl)-butyric acid] ruthenium (II); (2,2'- bipyridine) [bis-bis(1,2-diphenylphosphino)ethylene] 2-[3-(4-methyl-2,2'-bipyridine-4'-yl)propyl)-1,3-dioxolane osmium (II); bis (2,2'-bipyridine) [4-(4'-methyl-2,2'-bipyridine)-butylamine] ruthenium (II); bis (2,2'-bipyridine) [1-bromo-4 (4'-methyl-2,2'-bipyridine-4-yl)butane] ruthenium (II); and bis (2,2'-bipyridine)maleimidohexanoic acid, 4-methyl-2,2'-bipyridine-4'-butylamide ruthenium (II).

The function of the metal-containing ECL moiety in the present invention is to emit electromagnetic radiation as a result of introduction into the reaction system of electrochemical energy. In order to do this, the metal-containing ECL moiety must be capable of being stimulated to an excited energy state and also capable of emitting electromagnetic radiation, such as a photon of light, upon descending from that excited state. While not wishing to be bound by theoretical analysis of the mechanism of the metal-containing ECL moiety's participation, we believe that the ECL moiety is oxidized by the introduction of electrochemical energy into the reaction system and then, through interaction with a reductant derived from the species capable of forming a strong reducing agent, is converted to the excited state. This state is relatively unstable, and the metal-containing ECL moiety chelate quickly descends to a more stable state. In so doing, the ECL moiety gives off electromagnetic radiation, such as a photon of light.

Typically, in assaying operations the metal-containing ECL moiety is linked directly or through one or more other molecules to the analyte of interest or an analog thereof. Analogs of the analyte of interest, which can be natural or synthetic, are typically compounds which have binding properties comparable to the analyte, but can also be compounds of higher or lower binding capability. When the metal-containing ECL moiety is linked to the analyte or said analog through one or more other molecules, they are suitably a combination of one or more binding partners and/or one or more reactive components. Binding partners suitable for use in the present invention are well-known. Examples as antibodies, enzymes, nucleic acids, cofactors and receptors. The reactive components capable of binding with the analyte or its analog, and/or with a binding partner, are suitably a second antibody or a protein such as Protein A or Protein G, or avidin or biotin or another component known in the art to enter into binding reactions.

The amount of metal chelate or other metal-containing ECL moiety incorporated in accordance with the invention will vary from system to system. Generally, the amount of such ECL moiety utilized is that amount which is effective to result in the emission of a detectable, and if desired quantitatable, amount of electromagnetic radiation from the aforementioned composition. The detection and/or quantitation of an analyte of interest is typically made from a comparison of (i) the amount or wavelength of such electromagnetic radiation emitted by the ECL composition with (ii) data indicating the amount of electromagnetic radiation emitted when the concentration of the analyte of interest is known, such as in the form of a calibration curve. This, of course, assumes a homogeneous format. In the heterogeneous mode, a separation as discussed previously is carried out prior to ECL analysis.

As can be appreciated by one of ordinary skill in the art, the identity and amount of the metal-containing ECL moiety will vary from one system to another, depending upon prevailing conditions. The appropriate metal-containing ECL moiety, and sufficient amount thereof to obtain the desired result, can be determined empirically by those of ordinary skill in the art, once equipped with the teachings herein, without undue experimentation.

In a more specific embodiment a composition in accordance with the invention contains two or more different ECL moieties. Each of the ECL moieties can be induced to emit electromagnetic radiation of a wavelength different from the other moiety or moieties. In another embodiment of the invention, the ECL moieties can be species each of which

is induced to emit electromagnetic radiation by exposure to energy of a value different from the energy value(s) at which the other moiety or moieties emit radiation. In this manner it is possible to determine two or more different analytes of interest that may be present in the sample under examination.

Another essential feature of the present invention is the utilization of a species which can be oxidized to convert it to a highly reducing species. Once again, while not wishing to be bound by a theoretical explanation of reaction mechanism, it is believed that this species, such as a carboxylic acid, is oxidized by oxidized metal-containing ECL moiety (or other oxidized substance) present in the reaction system or by electrochemical energy. Illustratively, when the species is oxalate, it loses one electron, and then transforms into $CO_2$ and the radical ion $CO_2$-, the latter being a strong reducing agent. This agent interacts with the oxidized metal-containing ECL moiety and causes it to assume the excited state discussed above. The reductant derived from such species thus provides the necessary stimulus for converting the oxidized metal-containing ECL moiety to its excited state, from which electromagnetic radiation is emitted.

Typical examples of species which can be utilized in practicing the present invention are carboxylic acids. Generally, the species should be compatible with the environment in which it must function during analysis, i.e., compatible with an aqueous or nonaqueous environment as the case may be. Another consideration is that the species selected must form a reductant which, under prevailing conditions, is strong enough to reduce the oxidized metal-containing ECL moiety in the system leading to formation of the excited state.

Carboxylic acids which are advantageously utilized in the present invention are those having the formula

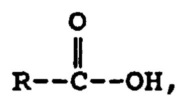

wherein R is $C_nH_{2n+1}$, $C_nH_{2n+1-y}X_y$, $R^1COOH$, $R^2CO$ or

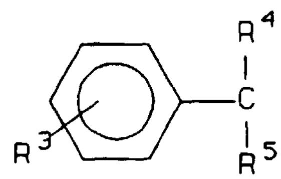

X being chlorine, bromine, fluorine or iodine; $R^1$ being $C_nH_{2n}$ or CHOH; $R^2$ being $C_nH_{2n+1}$,; $R^3$, $R^4$ and $R^5$ being hydrogen, $C_nH_{2n+1}$, phenyl, or substituted phenyl, each of $R^3$, $R^4$ and $R^5$ being the same, or not all of them being the same; n being from 0 to 20; and y being from 0 to 2n. Specifically, n can be from 2 to 10 and still more specifically, n can be from 4 to 6.

Specific examples of suitable carboxylic acids are oxalic acid, malonic acid, succinic acid, tartaric acid, citric acid, lactic acid, pyruvic acid and acids of the following formulae (phenyl being optionally substituted)

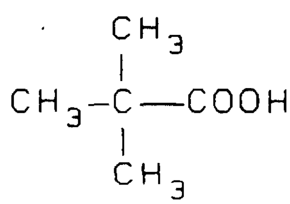 ;

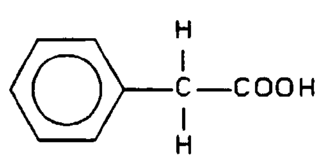 ;

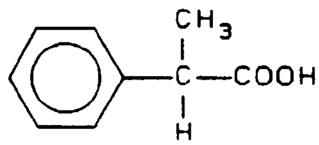 ;

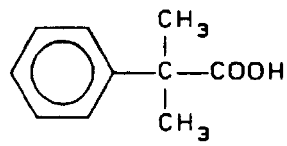 ;

and

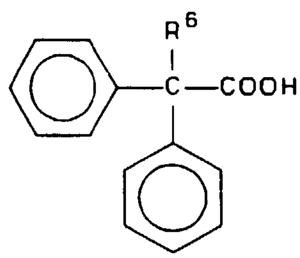 ;

wherein $R^6$ is H, $CH_3$ or phenyl. Oxalic acid ("oxalate") is especially preferred.

Esters of the aforementioned carboxylic acids are also suitable for practicing certain embodiments of the invention.

The species capable of forming strong reducing agents in accordance with the present invention work suitably well at a pH in a range of from 1 to 7, in some embodiments at a pH in a range of from 2- 6, and in certain embodiments at a pH in a range of from 3-6. Oxalic acid gives best results at a pH of 3-5.

Typically, the metal-containing ECL moiety utilized in the present invention is the reaction-limiting constituent. Accordingly, it is also typical that the species forming the strong reducing agent is provided in a stoichiometric excess in respect of the ECL moiety. Illustratively, such species is employed in a concentration of 5-200 mM, more specifically 5-50 mM and even more specifically 25-40 mM. In general, the amount of species forming a strong reducing agent employed is that which is sufficient to effect the transformation of the oxidized metal-containing ECL moiety into its excited state so that electromagnetic radiation emission occurs.

Those of ordinary skill in the art, equipped with the teachings herein, can determine empirically the identity and/ or amount of said species advantageously used for the particular system being analyzed, without undue experimentation.

The substance in the presence of which emitted electromagnetic radiation is increased is typically a compound of the formula

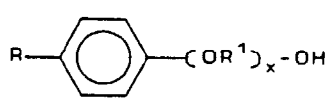 ;

wherein R is hydrogen or $C_nH_{2n+1}$, $R^1$ is $C_nH_{2n}$, x is 0 to 70 and n is from 1 to 20. Preferably, n is from 1 to 4. Specific examples are a substance available in commerce under the name Triton X-100®, of the formula

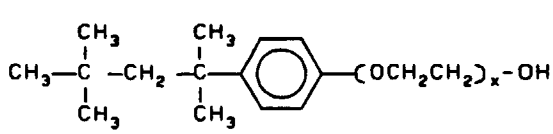 ;

wherein x is 9-10, and a substance available in commerce under the name Triton N-401 (NPE-40)®, of the formula

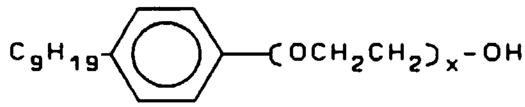

wherein x is 40. The substance is generally utilized in an amount sufficient so that in its presence the desired increase in emission of radiation occurs. Typically, the amount is .01% to 5.0%, more specifically 0.1% to 1.0%, v/v.

As noted above, the ECL moiety incorporated in accordance with the present invention is induced to emit electromagnetic radiation by stimulating it into an excited state. This is accomplished by exposing the composition in which the ECL moiety is incorporated to electrochemical energy. The potential at which oxidation of the ECL moiety occurs depends upon the chemical structure thereof, as well as upon factors such as the pH of the system and the nature of the electrode used to introduce electrochemical energy. It is well known to those of ordinary skill in the art how to determine the optimal potential and solution conditions for an ECL system.

Of course, in order to operate a system in which an electrode introduces electrochemical energy, it is necessary to provide an electrolyte in which the electrode is immersed and the ECL moiety, species from which the reducing agent is derived, and substance in the presence of which emitted radiation is increased, are contained. The electrolyte is a phase through which charge is carried by ions.

Generally, the electrolyte is in the liquid phase, and is a solution of one or more salts or other species in water, an organic liquid, or a mixture of water and an organic liquid. However, other forms of electrolyte are also useful in certain embodiments of the invention. For example, the electrolyte may be a dispersion of one or more substances in a fluid -- e.g., a liquid, a vapor, or a supercritical fluid -- or may be a solution of one or more substances in a vapor, a solid or a supercritical fluid.

The above-mentioned supercritical fluid is a dense gas maintained above its critical temperature, i.e., the temperature above which it cannot be liquified by any pressure. Supercritical fluids are less viscous and diffuse more readily then liquids. Examples of supercritical fluids which are useful in practicing the present invention are carbon dioxide, and certain alkanes such as methane, ethane and propane. The conditions at which supercritical behavior is exhibited are known in the art. See, for instance, Smith U.S. Patent No. 4,582,731 granted April 15, 1986. Utilization of supercritical fluids can be advantageous; for instance, in certain embodiments of the invention the solubility of various analytes of interest can be increased in a supercritical fluid. Also, the solubility of the ECL moiety and the species from which the strong reducing agent is derived can, in some embodiments, be more easily controlled in a supercritical fluid. Furthermore, sensitivity can in some cases be improved because of the higher diffusion coefficient of various species in these fluids.

In the case of compositions in accordance with the present invention which are aqueous, the electrolyte is aqueous, e.g., a solution of a salt in water. The salt can be a sodium salt or a potassium salt preferably, but incorporation of other cations is also suitable in certain embodi ments, as long as the cation does not interfere with the ECL interaction sequence. The salt's anion can be a phosphate, for example, but the use of other anions is also permissible in certain embodiments of the invention -- once again, as long as the selected anion does not interfere with the ECL interaction sequence.

The composition can also be nonaqueous. While supercritical fluids, vapors and solids can in certain instances be employed advantageously, it is far more typical to utilize an electrolyte comprising an organic liquid in a nonaqueous composition. Like the aqueous electrolytes, the nonaqueous electrolyte is also a phase through which charge is carried by ions. Normally, this means that a salt is dissolved in the organic liquid medium. Examples of suitable organic liquids are acetonitrile, toluene, dimethylsulfoxide (DMSO), dimethylformamide (DMF), methanol, ethanol, and mixtures of two or more of the foregoing. Illustratively, tetraalkylammonium salts, such as tetrabutylammonium tetrafluoroborate, are soluble in organic liquids and can be used with them to form nonaqueous electrolytes.

The electrolyte is, in certain embodiments of the invention, a buffered system. Phosphate buffers are often advantageous. Examples are an aqueous solution of sodium phosphate/sodium chloride, and an aqueous solution of sodium phosphate/sodium fluoride.

The formulation of electrolytes, including buffered systems, and a determination of suitable amounts of electrolyte for use in practicing the invention is within the skill of the art, once the practitioner is equipped with the teachings therein.

Utilization of the aforementioned materials in the present invention permits its practitioner to induce the emission of electromagnetic radiation from an ECL composition in accordance with the method embodiments of the invention.

The present ECL assay is accomplished by combining one or more metal-containing ECL moieties, one or more species from which a strong reducing agent is derived, one or more substances in the presence of which emitted electromagnetic radiation is increased, and a compatible electrolyte, to form a composition into which electrochemical energy can be introduced with the result that electromagnetic radiation is emitted. The composition is subjected to an amount of electrochemical energy which is effective to induce the composition to emit electromagnetic radiation.

By inducing the composition to emit electromagnetic radiation we mean generating an excited state of the ECL moiety in the composition, which excited ECL moiety gives off electromagnetic radiation -- for instance, luminesces at wavelengths from about 200 nanometers to about 900 nanometers at ambient temperatures. This excited state is achieved by oxidizing the ECL moiety. As previously noted, the potential at which the oxidation of the ECL moiety occurs depends upon its chemical structure as well as factors such as the pH of the composition and the nature of the electrode used. Once the ECL moiety is excited, it emits electromagnetic radiation upon interaction with the strong reductant discussed previously. Determination of the optimal potential and emission wavelength for an ECL composition is within the ordinary skill of the art once it is in possession of the teachings herein. The amount of electromagnetic radiation emitted by the ECL moiety as it descends from the excited state can be measured directly as an indication of the amount of analyte present.
Alternatively, the electromagnetic radiation emitted when the ECl moiety descends from the excited state can be utilized to trigger a detectable event (or step in a sequence of steps culminating in a detectable event) which is measured, rather than the radiation emitted by the ECL moiety itself.

Radiation emitted by the ECL composition is detected using suitable means in order to permit a qualitative or quantitative determination of the analyte of interest.

This determination can be made either as a continuous rate-based measurement, or as an accumulation of the ECL signal over a long period of time. For example, rate-based measurements are made with photomultiplier tubes, photodiodes or phototransistors to produce electric currents proportional in magnitude to the incident light intensity, or by using charge coupled devices, whereas examples of cumulative methods are the integration of rate-based data, and the use of photographic film to provide cumulative data directly.

The composition is formulated in order to obtain the desired pH, concentration of ECL moiety, concentration of species from which the strong reductant is derived, concentration of substance in the presence of which the emission of radiation increases, and electrolyte. In this connection, said metal-containing ECL moieties, species from which strong reducing agents are derived, substances and electrolytes, and suitable and preferred amounts and concentrations thereof in the composition, are as described elsewhere herein.

The composition can be made by combining its individual ingredients. However, it is often more advantageous to utilize one or more reagents containing a combination of various substances from which the composition is made. This measure facilitates the maintenance of uniformity in the compositions formulated according to the invention, which contributes to the reliability and repeatability achieved with practice of the invention.

Accordingly, a reagent suitable for formulation of the composition can comprise the substance in the presence of which emitted radiation is increased, in combination with any one or more of the metal-containing ECL moiety, the reductant-forming species and the electrolyte. Whichever reagent is chosen can be combined with the balance of the ingredients necessary to formulate the composition. One or more of those ingredients can also be contained in another reagent. For instance, a reagent comprising said substance, metal-containing ECL moiety and electrolyte in combination can be mixed with another reagent comprising said substance, reductant-forming species and electrolyte in combination to yield the desired composition.

The formation of the composition is suitably accomplished with the use of a kit comprising one or more reagent components necessary for the formulation step. Thus, the overall kit contains (i) a metal-containing ECL moiety, (ii) a reductant-forming species, (iii) an electrolyte and (iv) a substance in the presence of which emitted radiation is increased, all as described previously. An attractive aspect of packaging ingredients used to formulate the composition in kit form is that standardized ingredients, provided as one or more reagents for convenience, can be employed to improve the reliability and repeatability of practice of the invention. Use of reagents and other materials in kit form is additionally advantageous in that the possibility of degradation of the ingredients before use is minimized, since the kit formats utilized can be structured so as to avoid combinations in which any such degradation might occur.

Accordingly, the composition can be formulated from a kit in which any two or three members of the group consisting of the metal-containing ECL moiety, the reductant-forming species, the electrolyte and said substance can be included in a first separate component and the remaining member or members of the group in a second separate component, or in a plurality of different components. (The component(s) of the kit is or are typically kept separate by enclosing each in its own vial so as to eliminate cross-contamination prior to combination.) An alternative is a kit comprising a first separate component including any two or three members of the aforementioned group, and a second separate component including the remaining member or members of the group and one or more of the members included in the first group. Another alternative is a kit which comprises four separate components, each of which includes a different one of the four above-mentioned group members. In yet another format, the kit can comprise a first separate component including all four members of the group, and a second separate component including any one, two or three of the members of that group; said kit optionally comprises a second component containing one of the four group members, and further comprises a third separate component containing one, two or three (preferably two) group members and a fourth separate component containing one or two group members.

More specifically, in an advantageous embodiment, the first separate component of a kit includes the metal-containing ECL moiety, and the second separate component includes reductant-forming species, the electrolyte, and the substance in the presence of which emitted radiation is increased.

As mentioned above, with the present invention the emission of electromagnetic energy is brought about by exposing a composition as discussed above to an amount of electrochemical energy effective to induce such emission. Advantageously, the emission is induced by exposing the composition, and thus the metal-containing ECL moiety therein, to a voltammetric working electrode. The ECL reactive mixture is, accordingly, controllably triggered to emit light or other electromagnetic radiation by a voltage impressed on the working electrode at a particular time and in a particular manner effective to result in such generation of light or other form of electromagnetic radiation as is desired. The necessary voltage can be derived empirically by one of ordinary skill in the art, equipped with the teachings herein, without undue experimentation.

The use of the invention is further explicated in connection with the discussion of apparatus suitable for carrying it out as illustrated in Figures 1 and 2.

Figure 1 discloses an advantageous apparatus for generating electrochemiluminescence. However, the present methods are not limited to application with apparatus 10, but rather can be implemented with other types of apparatus including a working electrode or other triggering surface to provide electrochemical energy to trigger electrochemiluminescence. While the methods of the invention can be carried out in a static or flow-through mode, apparatus 10 is a flow-through cell, which provides distinct advantages for many types of ECL operation, for example, handling of many types of samples including binding assay samples.

Apparatus 10 includes an electrochemical cell 12, a light detection/measurement device 14, which can advantageously be a photomultiplier tube (PMT), photodiode, charge coupled device, photographic film or emulsion or the like, and a pump 16, which is advantageously a peristaltic pump, to provide for fluid transport to, through and from cell 12. Alternatively, a positive displacement pump may be used. A shutter mechanism 18 is provided between cell 12 and PMT 14 and is controllably operated to open only so far as to expose PMT 14 to cell 12 during periods of measurement of electrochemiluminescence. Shutter mechanism 18 can be closed, for example, during maintenance. Advantageously, included in apparatus 10 but not shown in Figure 1 (for purposes of simplicity and clarity) is a lightproof housing inside of which the various components of the apparatus can be disposed to shield PMT 14 from any external light during measurements of electrochemiluminescence.

Cell 12 itself includes a first mounting block 20 through which passes an inlet tube 22 and an outlet tube 24, advantageously constructed of stainless steel. Mounting block 20 has a first, outer surface 26 and a second, inner surface 28 defining one side of a sample-holding volume 30 in which cell 12 holds the cleaning and/or conditioning and/or measurement solutions during corresponding operations of apparatus 10. Inlet and outlet tubes 22, 24 pass through mounting block 20 from outer surface 26 to inner surface 28 and open into sample-holding volume 30. A second mounting block 32, advantageously constructed of stainless steel also has a first, outer surface 34 and a second, inner surface 36. second mounting block 32 is separated from first mounting block 20 by an annular spacer 38, advantageously constructed of Teflon or other noncontaminable material. Thus, outer surface 34 of mounting block 20 defines part of the second side of the sample-holding volume 30. Spacer 38 has an outer portion 40 and a central aperture 42, the inner edge 44 of which defines the sidewall of sample-holding volume 30. Outer portion 40 seals the inner surface 28 of first mounting block 20 to outer surface 34 of second mounting block 32 to prevent any solution from passing out from sample-holding volume 30 between the two surfaces 28, 34. Mounting block 32 further has a central aperture 46 in which a window 48 is seal-fitted to define the rest of the second side of sample-holding volume 30 as a continuation of outer surface 34. Window 48 is formed of a material which is substantially transparent at the wave length of ECL light generated by the system in sample-holding volume 30. Window 48 is therefore advantageously formed of glass, plastic, quartz or the like.

Inlet tube 22 intersects sample-holding volume 30 at a first end 50 thereof adjacent to spacer 38, and outlet tube 24 intersects sample-holding volume 30 at a second end 52 thereof adjacent to spacer 38. Combination of inlet tube 22, sample-holding volume 30 and outlet tube 24 thereby provides a continuous flow path for the narrow, substantially laminar flow of a solution to, through and from cell 12.

Mounted on inner surface 28 of first mounting block 20 is a working electrode system 54 which, in the illustrated embodiment, includes first and second working electrodes 56 and 58. In other embodiments, a single working electrode may advantageously be provided or only electrode 56 may be a working electrode. Working electrodes 56, 58 are where the electrochemical and ECL reactions of interest can take place. Working electrodes 56, 58 are solid voltammetric electrodes and therefore can advantageously be constructed of platinum, gold, carbon or other materials which are effective for this purpose. Wire connectors 60, 62 connected to working electrodes 56, 58 respectively, pass out through first mounting block 20.

Connectors, 60, 62 are both connected to a first, "working electrode" terminal 64 of a voltage control 66, illustrated in Figure 2. Voltage control 66 advantageously operates in the manner of a potentiostat to supply voltage signals to working electrodes 56, 58 and optionally to measure current flowing therefrom during measurement of electrochemiluminescence. Alternatively, connectors 60, 62 may be connected to separate terminals of voltage control 66 for individual operation.

The potentiostat operation of voltage control 66 is further effected through a counter electrode 68 and, optionally advantageously, a reference electrode 70. In the illustrated embodiment, mounting block 32 is made of stainless steel and counter electrode 68 consists in exposed surfaces 72, 74 of mounting block 32. Counter electrode 72, 74 and working electrodes 56, 58 provide the interface to impress the potential on the solution within sample-holding volume 30 which energizes the reactions of interest and triggers electrochemiluminescence in the sample and/or provides energy for cleaning and conditioning the surface of cell 12. Counter electrode 72, 74 is connected by a wire connector 76, to a second "counter electrode" terminal 78 of voltage control 66.

Reference electrode 70 provides a reference voltage to which the voltage applied by the working electrodes 56, 58 is referred, for example, + 1.2 volts versus reference. Reference electrode 70 is advantageously located in outlet tube 24 at a position 80 spaced from cell 12 and is connected through a wire connector 82 to a third "reference electrode" terminal 84 of voltage control 66. In the three electrode mode, current does not flow through reference electrode 70. Reference electrode 70 may be used in a three electrode mode of operation to provide a poised, known and stable voltage and is therefore advantageously constructed of silver/silver chloride (Ag/AgCl) or is a saturated calomel electrode (SCE). Voltage control 66 can also be operated in a two electrode mode using only working electrode 56 and electrode 58 as a counter/reference electrode. In this two electrode mode of operation, counter/reference electrode 58 is electrically connected to voltage control terminals 78 and 84 on voltage control 66. In this case, voltage control 66 operates essentially as a battery. Voltage control 66 applies voltage signals to working and counter electrodes 56 and 58 and optionally measures the current flowing through the respective electrodes. Reference electrode 70 may alternatively be a so-called "quasi-reference" electrode constructed of platinum, gold, stainless steel or other material, which provides a less stable voltage, but one that is measurable with respect to the solution in contact. In both the two and three electrode modes, the reference electrode 70 or 58 serves the purpose of providing a reference against which the voltage applied to the working electrode(s) is measured. The poised voltage reference is currently considered to be more advantageous. Voltage control 66 in its potentiostat operation controls various electrodes by providing a known voltage at working electrodes 56, 58 with respect to reference electrode 70 while measuring the current flow between working electrodes 56, 58 and counter electrodes 72, 74. Potentiostats for this purpose are well known, and the internal structure of voltage control 66 therefore suitably corresponds to any of the conventional, commercially available potentiostats which produce the above-mentioned functions, and so does not form a part of the present invention per se. Indeed, apparatus 10 can alternatively be constructed without an internal voltage control 66, and can be adapted to be connected to an external potentiostat which is separately controlled for providing required voltage signals to electrodes 56, 58, 72, 74 and 70. These voltage signals, applied in a specific matter as described below, provide repeatable initial conditions for the surfaces of working electrodes 56, 58 and advantageously for the surfaces of cell 12 as a whole, a feature which contributes significantly to improved processing in the measurement of electrochemiluminescence.

Pump 16 is advantageously positioned at outlet 24 to "pull" solution from a sample volume in the direction of arrow A into inlet tube 22. The solution will flow to inlet tube 22, sample-holding volume 30 and outlet tube 24, past reference electrode 70 and out in the direction of arrow B. Alternatively, pump 16 may be positioned at inlet 22 to "push" the solution through apparatus 10. Advantageously, this same flow path through inlet-tube 22, sample-holding volume 30 and outlet tube 24 is used for all solutions and fluids which pass through cell 12, whereby each fluid performs a hydrodynamic cleaning action in forcing the previous fluid out of cell 12. Pump 16 may be controlled to suspend its operation to hold a particular solution in cell 12 for any period of time.

The flow-through construction of apparatus 10 permits working electrodes to be impressed with a variable voltage to be held continuously at a preoperative potential while being continuously exposed to one or more solutions without exposing working electrodes 56, 58 (or counter and reference electrodes 72, 74, 70) to air. Exposure to air, which opens the circuit to the reference electrode 70, permits unknown, random voltage fluctuation which destroys the reporducibility of surface conditions on working electrodes 56, 58. The flow-through construction permits the rapid alternation between initializing steps, in which electrode system 54 is cleaned and conditioned, and measurement steps, in which one or more measurement waveforms or sweeps trigger electrochemiluminescence.

From the foregoing, it is evident that a composition comprising a metal-containing ECL moiety, a reductant-forming species, an electrolyte and a substance in the presence of which emitted radiation is increased in accordance with the invention is introduced into cell 12, and exposed to electrochemical energy, advantageously by impressing a suitable voltage on one or more electrodes of the system as described above (or other suitable system as can readily be derived by one of ordinary skill in the art when equipped with the teachings herein) to induce the desired electrochemiluminescence.

The amount of light or other electromagnetic radiation emitted by the reaction system in question is indicative of the presence or absence of an analyte, and, if it is present, in what amount. Thus, qualitative and quantitative analysis of a sample for an analyte of interest is enabled. In this connection, when the electromagnetic radiation emitted is light, that emission can be detected with a photometer which is connected to a computer, e.g., a personal computer. In that

computer, the signals received from the photometer are processed and, for instance, can either be displayed on a screen or be outputted via analog conversion to an appropriate strip-chart recorder.

A principal application of the present invention is the detection or quantitation of an analyte of interest in a given sample by ECL assay. As alluded to previously herein, a binding assay involving the ECL reaction of the present invention can be carried out in different formats.

In a first embodiment, a sample which the practitioner desires to investigate for the presence or absence of an analyte of interest is directly evaluated in order to determine whether or not electromagnetic radiation emission is changed (either decreased or increased) with reference to emission obtained from a comparable sample in which none of the ECL moiety present is linked, either directly or through one or more other molecules, to analyte of interest or an analog thereof. In a second embodiment, detection and, if the analyte of interest is present quantitiation thereof, can be accomplished by taking any steps necessary to formulate an ECL composition in accordance with the present invention from the sample, exposing the composition to electrochemical energy in accordance with the present invention, and then comparing the amount of electromagnetic radiation emitted with the electromagnetic radiation emissions from systems containing various known amount of the analyte of interest. An appropriate change in emission with the sample being investigated signals the presence and amount of the analyte.

The use of the invention can be applied in a variety of assay formats. Thus the invention may be used in homogeneous or heterogeneous assay formats, and may be used in all assay procedures known in the art, including forward and reverse assays, competition assays, immunometric assays, sandwich assays, and hybridoma screening assays.

As described in EP-A-0 446 245, it is desirable, in performing assays disclosed herein, to incorporate particles in the assay composition or system. Binding of such a component, which in turn is linked to an ECL moiety, to the particles greatly modulates the intensity of the ECL signal generated by the ECL moiety, thereby providing a means of monitoring the specific binding reaction of the assay composition or system. Further information on this topic is set forth in the above-mentioned European patent publication.

For example, a useful class of homogeneous binding assays provided by the present invention involves exposing a solution of the ECL moiety containing the analyte of interest to an electrode. ECL moiety which cannot gain access to the surface of the electrode will not be detected. This can occur, for example, if the ECL moiety is bound directly or indirectly to the surface of the reaction vessel into which the electrode is placed, or if the ECL moiety is buried deep in the interior of the specific complex, such as within an antigen-antibody complex, or if the electrode itself is coated with a layer through which ECL moiety can pass but ECL moiety linked (directly or indirectly) to the analyte of interest or its analog cannot pass. In addition, it should be possible to coat the surface of an electrode with antibodies, so that only antigen linked diretly or through one or more other molecules to the ECL moiety and bound to the immobilized antibodies can obtain access to the electrode and thereby be determined.

Competitive binding methods can be used in accordance with the invention to determine the presence of an analyte of interest. Typically, the analyte and the ECL moiety bind competitively to a chemical and biological material. The material is contacted with the ECL moiety and analyte under suitable conditions so as to form a suitable composition. The ECL moiety is induced to emit electromagnetic radiation by exposing the composition to electrochemical energy. The presence of the analyte of interest is determined by detecting the amount of electromagnetic radiation emitted by the composition.

In competitive binding assays, the analyte of interest and an analog thereof linked directly or through one or more other molecules to an ECL moiety can be any substances capable of participating in formation of a specific complex with a complementary material, such as for example, whole cells, subcellular particles, nucleic acids, polysaccharides, proteins, glycoproteins, lipoproteins, lipopolysaccharides, peptides, polypeptides, cellular metabolites, hormones, pharmacological agents, tranquilizers, barbituates, alkaloids, steroids, vitamins, amino acids, sugars and nonbiological polymers. Of particular interest are antibody-antigen based methods. These methods are analogous to the well known radioimmunoassay, wherein an analyte of interest is detected when it displaces a radioactive analogue of the analyte from an antibody. The many variations on radioimmunoassay known to the art can, in principle, be used to advantage by employing ECL moieties according to the present invention in place of radioactively labelled compounds.

The use according to the present invention can also be employed in binding assays used in a competition format, where the ECL moiety is linked directly or through one or more other molecules to added analyte of interest. The binding partner is capable of specifically binding with the analyte of interest or the added analyte of interest which is linked to the ECL moiety. The analyte of interest and the added analyte of interest are suitably an antigen.

Alternatively, the binding partner is a primary binding partner of the analyte of interest. The assay sample contains the ECL moiety linked directly or through one or more other molecules to added analyte of interest. The binding partner is bound to suitable particles in the sample, and the particles are therefore capable of specifically binding with the analyte of interest or the added analyte of interest linked to the ECL moiety. Here also, the analyte of interest and the added analyte of interest are typically an antigen.

The invention can also be employed in an assay of immunometric format. The ECL moiety is linked to a binding partner of the analyte of interest. The analyte or an analog thereof is bound to a surface and accordingly the surface

is capable of specifically binding with the binding partner. The surface can be the surface of a particle, membrane, strip, tube, etc. The analyte of interest can be an antigen.

Alternatively, the binding partner is a primary binding partner of the analyte of interest. A binding partner of the primary binding partner is a substance linked to the ECL moiety. Analyte or an analog thereof is bound to a surface and accordingly the surface is capable of specifically binding with the primary binding partner. The secondary binding partner linked to the ECL moiety specifically binds the primary binding partner. The analyte of interest is typically an antigen.

The invention can be used, for example, in sandwich assays as well. The analyte of interest can be an antigen. A substance linked to the ECL moiety is a binding partner of the analyte of interest. A binding partner not linked to the ECL moiety is bound to a surface and accordingly the surface is capable of binding to the analyte of interest.

Alternatively, the binding partner may be primary binding partner (BP-1) of the analyte of interest. A secondary binding partner of the primary binding partner is a substance linked to the ECL moiety. The analyte of interest can be an antigen. Another primary binding partner (BP-2) which is not recognized by the secondary binding partner is bound to the surface and accordingly the surface is capable of binding to the analyte of interest. The surface and primary binding partner (BP-1) are capable of specifically binding the antigen and the secondary binding partner linked to the ECL moiety is capable of specifically binding the primary binding partner (BP-1). Also, the binding partner can be a primary binding partner (BP-1) of the analyte of interest. BP-1 is linked to the ECL moiety. Another primary binding partner (BP-1') which is different from BP-1 and binds the analyte of interest is used. A secondary binding partner of the primary binding partner BP-1' is bound to a surface and accordingly the surface is capable of binding the complex of analyte BP-1 and BP-1'.

The uses of the invention are advantageously employed in nonseparation binding assays for use in hybridoma screening assay formats. The analyte of interest is a monoclonal antibody directed against a particular antigen. A binding partner of the analyte of interest is linked to the ECL moiety. Antigen is bound to a surface and accordingly the surface is capable of specifically binding with the analyte. The monoclonal antibody specifically binds the surface and the binding partner which is part of the ECL moiety specifically binds the monoclonal antibody.

Advantageously, the binding partner in the ECL moiety capable of specifically binding the monoclonal antibody is a polyclonal antibody, a monoclonal antibody, protein A, or protein G. In addition, that binding partner may be avidin, which can bind to a biotin-modified analyte or binding partner.

Alternatively, the binding partner is a primary binding partner of the analyte of interest. A binding partner of the primary binding partner is linked to the ECL moiety. The analyte of interest is a monoclonal antibody directed against an antigen. Antigen is bound to a surface and accordingly the surface is capable of specifically binding with the monoclonal antibody. The monoclonal antibody specifically binds the surface, the primary binding partner specifically binds the monoclonal antibody, and the secondary binding partner in the ECL moiety specifically binds the primary binding partner.

The invention is further described and illustrated in the following examples.

Example 1

Electrochemiluminescence measurements were performed with the following apparatus:

A cell was made from a small sample test tube and electrodes which could be dipped into a sample. A Berthold luminometer (model LB9500T, equipped with a Hamamatsu R374 photomultiplier tube (PMT) at 1375 volts) having a chemiluminescence reagent addition arm that, when lowered, formed a light-tight seal over the small test tube that contained the sample, was positioned in front of the photomultiplier tube (PMT) for light collection. The reagent addition arm was modified to hold the electrodes and electrode connection wires to a potentiostat (Princeton Applied Research--model PAR 173 potentiostat and PAR 175 universal programmer) that dipped into the sample solution when this arm was lowered. The electrodes consisted of two parallel platinum gauze electrodes (1cm$^2$ geometrical area) separated by about 2mm. When in place, the electrodes faced parallel to the PMT -- the one closest to the PMT was the "working electrode" while the other formed the "counter electrode." A silver wire, serving as the "reference electrode," was positioned between the two Pt gauzes.

Materials

- Tris (2,2'-bipyridly) ruthenium chloride hexahydrate (Aldrich, Cat. No. 22475-8) dissolved in water to give a 1mM solution (the "TAG solution");
- Crystalline oxalic acid (Sigma 0-0505) dissolved in water to give a 1M solution;
- Phosphate buffer (0.10 M, pH 6.0);
- Triton-X-100- (Sigma T-6878).

Procedure

A tube containing about 1 ml of sample was positioned in the modified Berthold luminometer. The arm holding the electrode was lowered fully, such that the electrode was now positioned within the sample solution. Electrochemiluminescence was triggered with the potentiostat applying a sweep voltage from +0.7 V to +1.3V at 10mV/sec. All voltages were referenced to the silver reference electrode. Photons generated were integrated on the Berthold equipment for 30 sec. After each measurement, the electrode was removed, rinsed with water, wiped dry with a Kimwipe, and positioned into the modified arm for the next sample measurement.

0.9 ml of phosphate buffer was pipetted into (12x75mm) plastic tubes (Berthold tubes). Then, in one case 20 ul of 1M oxalic acid were also added. In another case 20 µl of 1 M oxalic acid and 10 ml of 1mM TAG solution were also added. In yet another case 20µl of 1M oxalic acid, 10 µl of 1 mM TAG solution and 10 µl of Triton X-100 were also added. Electrochemiluminescence measurements were performed on each combination, as described above.

Results

The results were as set forth in the following table:

TABLE I

| Substance (s) Measured | Electroluminescence |
|---|---|
| buffer, oxalic acid | 7800 counts |
| buffer, oxalic acid, TAG solution | 16,000; 16,500 counts |
| buffer, oxalic acid, TAG solution, Triton X-100 | 661,000; 579,000 counts |

Example 2

Additional electrochemiluminescence measurements were performed in the apparatus shown in Figure 1.

Materials

A buffer containing the precusor of a strong reducing agent and Triton X-100 was prepared by dissolving 15.599 g of $NaH_2PO_4$ and 3.12 g of oxalic acid in approximately 900 ml of deionized water. The pH was adjusted to 4.0 by dropwise addition of 50% NaOH. Then 10 ml of Triton X-100 and distilled water were added to give a 100 ml solution of 0.1 M phosphate, 40 mM oxalate and 1.0% (v/v) Triton X-100 (pH 4.0)

The following standard solutions were prepared by dilution from a stock solution of 100 nM tris (2,2'-bipyridyl) ruthenium chloride hexahydrate ("TAG"):

a. 100 pM
b. 1.0 nM
c. 10.0 nM

Procedure (Voltammograms and Emission Profile)

Using the flow through system which has been described in connection with Figure 1, measurements of electrochemiluminescence were taken with the following procedure:

a. introduce buffer and step potential to 1.1V;
b. introduce 0.5 ml sample;
c. conduct a voltage sweep from 1.1V to 2.5V (during which time a voltammogram and electrochemiluminescence were recorded), and then to -1.0V and back to 1.1V (100 mV/ sec).

Results

Electrochemiluminescence readings were taken and the results are tabulated in Table II.

TABLE II

| (No. of Counts during sweep) | | | | |
|---|---|---|---|---|
| | Background | 100 pM TAG | 1 nM TAG | 10 nM TAG |
| | 15 | 29 | 176 | 1556 |
| | 17 | 32 | 175 | 1525 |
| | 18 | 30 | 177 | 1525 |
| | 15 | 27 | 172 | 1550 |
| | 17 | 30 | 174 | 1525 |
| (No. of Counts during sweep - Repeat) | | | | |
| | Background | 100 pM TAG | 1 nM TAG | 10 nM TAG |
| | 15 | 33 | 184 | 1700 |
| | 16 | 33 | 186 | 1700 |
| | 17 | 35 | 186 | 1710 |
| | 16 | 33 | 188 | 1700 |
| | 16 | 35 | 186 | 1720 |
| Average | 16.0 | 33.3 | 186.0 | 1706.0 |
| Standard Deviation | 0.7 | 1.1 | 1.4 | 8.9 |
| % Coefficient of Variation (CV) | 4.4 | 3.2 | 0.8 | 0.5 |

Using an average of five points from the repeat readings shown in the lower half of Table II, a calibration curve was generated as is shown in Figure 3. The resulting linear curve is described by the equation y = 17 + 168.9x and the slope is the coefficient of the abscissa, i.e., 168.9. The lowest limit of detection (DL) was calculated in accordance with the following equation:

$$DL = C_{min}TAG = 2.5\%\ CV\ I_{BL}/100R$$

wherein $C_{min}TAG$ is the minimum concentration of the TAG (here, tris (2,2'-bibyridyl) ruthenium chloride hexahydrate), %CV is the percent coefficient of variation, $I_{BL}$ is the intensity of the blank (background) and R is the slope of the calibration curve. Substituting values for the various coefficients in the above equation, the lowest limit of detection was calculated as approximately 10.4 pM. It should be noted that this detection limit is 1000 times lower than the detection limit reported in the literature (10,000 pM; Ege, D., et al., J. Anal. Chem. 1984, 56, 2413).

Example 3

The Ru(II)-Compound III Conjugate described in Example 33 of WO 87/06706 was diluted to a final concentration of 150 nM using 0.1M phosphate buffer, pH 6.0, containing 0.35 M sodium fluoride (PBF Buffer). Monoclonal antibody (clone number 9-49, ascites lot number WO399, Cat. No. 046) specific for theophylline was obtained from Kallestad Laboratories, Inc. (Chaska, MN). The monoclonal antibody was diluted to different concentration using PBF Buffer (between 21.9 micrograms of protein/ml to 700 micrograms/ml).

Another monoclonal antibody (control MAB) that was not reactive with theophylline was obtained from Sigma (St. Louis, MO) and was diluted to different concentrations between 21.9 micrograms of protein/ml to 700 micrograms/ml using PBF Buffer. A standard solution of theophylline was prepared using theophylline obtained from Aldrich Chemical Co., (Milwaukee, WI, cat number 26-140-8, M.W. 180.17). Theophylline was dissolved in PBF Buffer to give a final concentration of 75 micromolar and was diluted with PBF Buffer to 6 micromolar for use in assays. Prior to making electrochemiluminescence measurements a solution containing 250 mM oxalic acid and 5% (v/v) Triton-X 100 (ECL solution) was added to the reaction mixture. Measurements were made using a Berthold luminometer that was modified to allow the placement of two platinum gauze electrodes into the test tube containing the reaction solution. The electrodes were connected to a potentiostat and the electrochemiluminescence measurement was made by sweeping an applied potential across the electrodes from 1.5 to 2.5 volts at a scan rate of 50 mV/sec. The Berthold luminometer used for the measurement had a high gain, red sensitive photomultiplier tube. The luminometer output to the recorder was adjusted to $10^5$ counts/volt. The measurements were recorded on an X-Y-Y' recorder and the peak height was

used as the measurement of electrochemiluminescence. The electrodes were cleaned between measurements by (a) rinsing with a buffer at pH 4.2 containing 0.1 M phosphate, 0.1 M citrate, 0.025 M oxalic acid, and 1% Triton X-100, (b) pulsing the electrodes in this solution between +2.2 to -2.2 volts for 60 sec, and (c) then applying +2.2 volts for 10 seconds. Next the electrodes were removed from this solution, rinsed in distilled water and wiped dry. The experiment was carried out as outlined in Table III.

A solution of control monoclonal antibodies, antibodies to theophylline or PBF Buffer was added to a set of test tubes (Step 1). To the tubes, a solution of theophylline or PBF Buffer was added (Step 2). The solutions were mixed by briefly shaking the test tubes and allowed to react for 25 min at room temperatue. Then a solution of Ru(II)-Compound III Conjugate was added to the tubes (Step 3). The test tubes were shaken and kept at room temperature for 15 min. Finally, 100 microliters of the ECL solution was added to each tube and electrochemiluminescence was measured as described above. The results are listed in Table IV.

Table III

| **Experimental Design for Studying the Effect of Antibody-Ru(II)-Compound III Conjugate Interactions on Electrochemiluminescence** | | |
|---|---|---|
| **Step I<br>100 microliters of:** | **Step 2<br>200 microliters of:** | **Step 3<br>100 microliters of:** |
| A. Control mono- clonal antibody (2.19 micrograms to 70 micrograms)<br>or | Buffer | Ru(II)Compound III Conjugate |
| B. Anti-theophylline antibody (2.19 micrograms to 70 micrograms)<br>or | Buffer or Theophylline | Ru(II)-Compound III Conjugate |
| C. PBF Buffer | Buffer | Ru(II)-Compound III Conjugate or Buffer |

TABLE IV

| **Effect of Antibody and Theophylline on Electrochemiluminescence of Ru(II)-Compound III Conjugate** | | | |
|---|---|---|---|
| **Antibody Protein Concentration (micrograms/tube)** | **Control MAB + Ru(II) Compound III** | **Anti-theophylline MAB + Ru(II)-Compound III** | **Anti-theophylline MAB + Theophylline Ru(II)-Compound III** |
| **Luminescence Measurement** | | | |
| 2.19 | 55,000 | 40,000 | 43,000 |
| | 55,000 | 41,000 | 57,000 |
| 4.38 | 57,000 | 22,500 | 37,000 |
| | 57,000 | 25,000 | 36,000 |
| 8.75 | 53,000 | 20,000 | 33,500 |
| | 50,000 | 22,000 | 30,500 |
| 35.0 | 43,000 | 13,500 | 17,500 |
| | 41,000 | 14,000 | 16,000 |
| 70.0 | 42,000 | 11,000 | 11,000 |
| | 37,500 | 12,000 | 12,500 |

The electrochemiluminescence of duplicate samples was measured as described above. The electrochemiluminescence of Ru(II)-Compound III Conjugate used in the above study was 57,200 when measured in buffer without the addition of antibody. The background for the buffer mixture was 5750.

The data show that a monoclonal antibody which specifically recognizes theophylline, when contacted with an analog of theophylline to which a ruthenium compound is attached, e.g., Ru(II)-Compound III, will decrease the electrochemiluminescence. The decrease in electrochemiluminescence is proportional to the antibody concentration when

the Ru(II)-Compound III Conjugate concentration is held constant. When an antibody is used which does not react with theophylline, only a slight decrease in the electrochemiluminescence is seen at the highest concentration of antibody.

The data also show that when theophylline is contacted with the anti-theophylline antibody and then the Ru(II)-Compound III Conjugate is added to the mixture, the amount of electrochemiluminescence is greater. This demonstrates that theophylline competes for the binding of antibody to Ru(II)-Compound III Conjugate resulting in a greater amount of Ru(II)-Compound III Conjugate when can generate electrochemiluminescence.

Example 4

Based on the results described in Example 3, a homogeneous immunoassay for theophylline was developed using antibody to theophylline and the Ru(II)-Compound III Conjugate mentioned in Example 3 in a competitive binding format. The materials used were as described in Example 3 except the PBF buffer was 0.1M phosphate buffer, pH 6.0, containing 0.1M sodium fluoride. For this assay, a specific concentration of monoclonal antibody to theophylline was chosen. The antibody concentration was 55 micrograms/ml. The Ru(II)-Compound III Conjugate concentration was adjusted to 175nM. Theophylline was added to human serum to give final concentrations of 2.5, 5, 10, 20 and 40 micrograms of theophylline/ml of serum.

The assay was performed by adding 10 microliters of serum to 290 microliters of anti-theophylline monoclonal antibody and holding the solution at room temperature for 25 min. Then 100 microliters Ru(II)-Compound III Conjugate were added to each tube to give a final concentration of 35 nM and holding this solution at room temperature for 15 min. 100 microliters of the ECL solution described in Example 3 were then added to each tube and electrochemiluminescent properties of the solutions were measured as previously described using a sweep mode for 1.5 volts to 2.5 volts at 50 mV/sec. The data are shown in Figure 4 and demonstrate that there is a correlation between the concentration of theophylline in a serum sample and the amount of electrochemiluminescence that is emitted by the reaction mixture. This observation demonstrates that it is possible to develop an assay for theophylline.

Based on these results, one skilled in the art would be able to develop a homogeneous electrochemiluminescence immunoassay for detecting and quantifying an analyte of interest in a biological matrix.

Example 5

The concentration of theophylline in different types of serum samples was determined using a homogeneous ECL immunoassay. The format for the assay was a competitive binding assay using a monoclonal antibody specific for theophylline and the Ru(II)-Compound III Conjugate mentioned in Example 3. The reagents and methods for electrochemiiuminescence are described in Example 3.

For purposes of comparison, a fluorescence polarization assay was also performed. The fluorescence polarization assay used to measure the concentration of theophylline in the different serum samples was carried out using an automated TDX instrument from Abbott Laboratories (North Chicago, IL). Hemolyzed, lipemic, icteric and normal sera were used in the assays and data for the abnormal sera are listed in Table V below.

TABLE V

| Homogeneous Theophylline Assay Characteristics of Potentially Problematic Sera | | |
|---|---|---|
| **Serum** | **Factor Concentration** | **Normal Range** |
| Hemolyzed | 12.4 mg/dl hemoglobin | 0-3.2 mg/dl |
| Lipemic | 405 mg/dl triglycerides | 10-190 mg/dl |
| | 196 mg/dl cholesterol | 120-200 mg/dl |
| Icteric | 10 mg/dl bilirubin | 0-1.5 mg/dl |

Different amounts of theophylline were added to the serum samples to give final concentrations between 2.5 micrograms theophylline/ml and 40 micrograms theophylline/ml. The results for the homogeneous ECL immunoassay are displayed in Figure 5.

Each serum sample was also analyzed for the concentration of theophylline by a fluorescence polarization assay. The concentration of theophylline measured by the homogeneous electrochemiluminescence immunoassay and the fluorescence polarization assay were compared. The data were plotted as a scattergram and are shown in Figures 6A-D. The data points were analyzed by linear regression and the correlation coefficients were calculated. The analysis emonstrates an excellent correlation between the two assays. The correlation coefficients (r) were between 0.98 and 1.00. The slopes of the curves for normal, hemolyzed, and lipemic serum samples were between 0.8 and 1.2, demonstrating excellent recovery of theophylline from these serum samples.

Although the electrochemiluminescence emitted by the icteric serum samples containing theophylline was higher than for the other serum samples, it was proportionally higher at each theophylline concentration. This can be seen in Figure 6D. The correlation coefficient is 1.00 for the data points comparing electrochemiluminescence and fluorescence polarization; however, the slope is 2.14, demonstrating higher recovery for the theophylline in the icteric serum sample.

Based on these results, the concentration of theophylline in an icteric sample may be determined by establishing a standard curve for the sample by adding known amounts of the Ru(II)-Compound III Conjugate to aliquots of the icteric serum. These data demonstrate that a homogeneous ECL immunoassay may be used to measure the concentration of theophylline present in serum samples containing abnormal levels of hemoglobin, lipid and bilirubin.

A homogeneous ECL immunoassay offers advantages over a fluorescence polarization method because of the versatility of ECL detection, e.g., more sensitive detection at higher concentrations of biological molecules.

A homogenous ECL immunoassay offers further advantages over a fluorescence polarization method because no incident light source is required, electrical excitation being the only requirement for efficient light-generation. Consequently, sophisticated optics are not necessary. Since the measurement principle is purely specific photon emission induced by electrochemical stimulation, the sensitivity of the system is potentially much greater than fluorescence polarization and a wider dynamic range will be achievable. Also, measurement of a much greater variety of analytes is possible with a homogeneous ECL immunoassay than is provided by the fluorescence polarization technique, due to the selective modulation of electrochemiluminesence by biomolecular recognition events, e.g., antibody-antigen interactions.

Based on these results, one skilled in the art would know that homogeneous ECL immunoassays for detecting other analyses of interest in abnormal serum samples may be developed.

Example 6

10 mg of solid digoxin were dissolved in 10 ml of DMSO:$H_2O$ (8:2), to give a digoxin concentration of 1 mg/ml (hereinafter "Stock Standard").

Working standards were prepared from the Stock Standard to the following concentrations in 0.15 M phosphate buffer, pH 7.0, containing 0.1% BSA and 0.15 M NaF (hereinafter "ECL buffer"): 80 ng/ml, 40 ng/ml, 20 ng/ml, 10 ng/ml, 5 ng/ml and 0 ng/ml.

75 microliters of anti-digoxin-Compound I conjugate described in WO 87/06706 (diluted 1:90) and 75 microliters of each standard were pipetted into a glass tube, mixed on a vortex and incubated at room temperature for 20 min.

50 microliters of prewashed oubain-BSA-Biomag particles were added to each tube, mixed on a vortex and incubated at room temperature for 5 min. Biomag particles were separated and supernatant was transferred to a separate tube.

100 microliters of supernatant were mixed with 400 microliters of 0.125 M potassium phosphate 0.125 M citric acid; 32 mM oxalic acid; 1.25% Triton X-100 in a tube.

The sample was placed into a Berthold instrument and the electrochemiluminescence was measured as previously described except the procedure was modified by stepping the applied potential from open circuit to 2.2V and integrating the photon counts for 10 sec.

The electrode was cleaned between measurements using photphate-citrate buffer as follows:

(a) Pulse electrode using 3 sec intervals alternating between -2.2V and +2.2V for 1 min.
(b) Poise the electrode at +2.2V for 10 seconds.
(c) Rinse electrode with deionized water and blot dry.

The results are shown in Figure 7.

**Claims**

**1.** The use of a compound or compounds of the formula

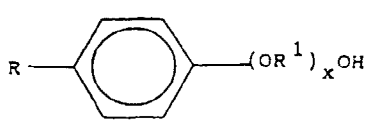

where R is hydrogen or $C_nH_{2n+1}$, $R^1$ is $C_nH_{2n}$, x is 0 to 70 and n is from 1 to 20, as an electromagnetic radiation

enhancer in a composition suitable for use in an ECL assay and comprising

(a) a metal-containing ECL moiety which, when oxidized by exposure to an effective amount of electrochemical energy, is capable of being converted to an excited state from which electromagnetic radiation is emitted upon exposure of the excited ECL moiety to conditions sufficient to induce said emission;
(b) a species, which, when oxidized, forms a strong reducing agent;
(c) an electrolyte capable of functioning as a medium in which said ECL moiety and said species can be oxidized.

**2.** The use as claimed in claim 1, wherein said ECL moiety is a metal chelate.

**3.** The use as claimed in claim 2, wherein the metal of said metal chelate is a transition metal or a rare earth metal.

**4.** The use as claimed in claim 3 wherein the metal of said metal chelate is ruthenium, osmium, rhenium, iridium, rhodium, platinum, indium, palladium, molybdenum, technetium, copper, chromium, or tungsten.

**5.** The use as claimed in any one of claims 1 to 4 wherein component (b) is a carboxylic acid.

**6.** The use as claimed in claim 5 wherein the carboxylic acid is oxalic, tartaric, citric, lactic, malonic, gluconic, or pyruvic acid.

**7.** The use as claimed in any one of claims 1 to 6 wherein said species forming a reducing agent is present in stoichiometric excess in respect of the ECL moiety.

**8.** The use as claimed in claim 5, claim 6 or claim 7 wherein the carboxylic acid is present in a concentration of 5-50mM.

**9.** The use as claimed in any preceding claim wherein said electrolyte is aqueous.

**10.** The use as claimed in claim 9 which includes an electrolyte comprising a salt dissolved in water.

**11.** The use as claimed in claim 9 or claim 10 which includes an electrolyte comprising water and an organic liquid miscible therewith.

**12.** The use as claimed in any one of claims 9 to 11 wherein the pH is 1-7.

**13.** The use as claimed in claim 12 wherein the pH is 2-6.

**14.** The use as claimed in any one of claims 1 to 8 wherein said electrolyte is nonaqueous.

**15.** The use as claimed in any one of claims 1 to 14 which includes an electrolyte comprising an organic liquid.

**16.** The use as claimed in claim 15 which includes an electrolyte comprising acetonitrile, DMSO, DMF, methanol, ethanol, or a mixture of two or more thereof.

**17.** The use as claimed in any one of claims 1 to 16 which includes an electrolyte comprising a phosphate buffer.

**18.** The use as claimed in any one of claims 1 to 16 wherein in the formula

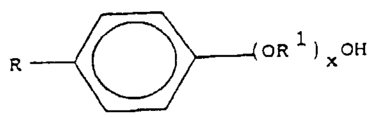

x is from 9 to 10.

**19.** The use as claimed in claim 4 wherein the metal chelate is a ruthenium-containing chelate, the species forming a strong reducing agent is oxalate, the electrolyte comprises a phosphate buffer and the compound of formula

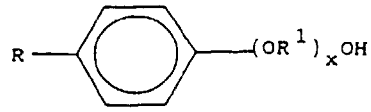

is Triton X-100®.

**20.** The use as claimed in any preceding claim wherein the compound of formula

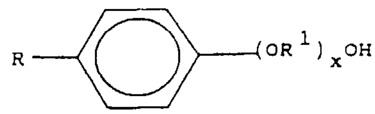

is incorporated in an amount of 0.1 to 1.0 percent wt./vol.

**21.** The use as claimed in any preceding claim wherein the composition is in the form of a kit, said kit comprising at least one separate component including one or more members of the group consisting of components (a), (b) and (c) and said compound.

**22.** The use as claimed in claim 21 in which the kit comprises a first separate component including any two members of said group, and a second separate component including the remaining members of said group.

**23.** The use as claimed in claim 22 wherein said first separate component contains said ECL moiety (a) and said electrolyte (c), and said second separate component contains said species (b) and said compound.

**24.** The use as claimed in claim 21 wherein the kit comprises a first separate component which includes any two members of said group and a second separate component which includes the remaining members of said group and also one of the members of the group which is included in said first component.

**25.** The use as claimed in claim 24 wherein said first separate component includes said species (b) and said electrolyte (c), and said second separate component includes said ECL moiety (a) and said compound.

**26.** The use as claimed in claim 21 which comprises four separate components, each of which includes a different one of the four members of said group.

**27.** The use as claimed in claim 21 which comprises a first separate component including all four members of said group, and a second separate component including any one, two, or three of the members of said group.

**28.** The use as claimed in claim 27 wherein said second separate component includes one of the four members of said group, and said kit further comprises a third separate component including one, two, or three members of said group, and a fourth separate component including one or two members of said group.

**29.** The use as claimed in claim 22 wherein said first separate component includes said ECL moiety (a) and said species (b), and said second separate component includes said electrolyte (c) and said compound.

**30.** The use as claimed in claim 22 wherein said first separate component includes said ECL moiety (a), and said second separate component includes said species (b), said electrolyte (c), and said compound.

**31.** The use as claimed in claim 21 wherein the kit comprises a first separate component including at least two members of said group and a second separate component including the remaining member or members of said group.

**32.** The use as claimed in any one of claims 22 to 31 wherein each second separate component is aqueous.

**33.** The use as claimed in any one of claims 21 to 32 wherein the kit is adapted to detect or quantitate an analyte of

interest by ECL assay, said kit further comprising at least one further substance selected from (i) an additional analyte of interest or an analog of the analyte of interest, (ii) a binding partner of the analyte of interest or its said analog, and (iii) a reactive component capable of binding the substance (i) or (ii), said further substance being contained in an additional separate component included within the kit, or in a component of said kit containing one or more of the members of said group.

**34.** The use as claimed in claim 33 wherein the kit is adapted for detection of an analyte selected from a whole cell or surface antigen, subcellular particle, virus, prion, viroid, antibody, antigen, hapten, fatty acid, nucleic acid, protein, lipoprotein, polysaccharide, lipopolysaccharide, glycoprotein, peptide, polypeptide, cellular metabolite, hormone, pharmacological agent, nonbiological polymer, synthetic organic molecule, organometallic molecule, tranquillizer, barbiturate, alkaloid, steroid, vitamin, amino acid, sugar, lectin, recombinant or derived protein, biotin, avidin, streptavidin, or inorganic molecule.

**35.** The use as claimed in claim 33 or claim 34 in which the kit is adapted for detection of an analyte specimen in a concentration of from $10^{-3}$ to $10^{-18}$ molar.

**36.** The use of a compound or compounds of the formula

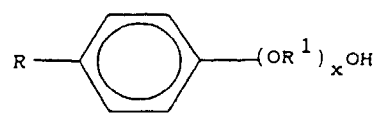

where R is hydrogen or $C_nH_{2n+1}$, $R^1$ is $C_nH_{2n}$, x is 0 to 70 and n is from 1 to 20, as an electromagnetic radiation enhancer in a method of generating emission of electromagnetic radiation which comprises the steps of

(i) forming a composition as specified in any one of claims 1 to 20;
(ii) exposing the composition under suitable conditions to an amount of electrochemical energy effective to induce the composition to emit electromagnetic energy; and
(iii) detecting the emitted electromagnetic radiation;

said compound being included in said composition.

**37.** The use as claimed in claim 36 wherein the composition is formed from a kit as claimed in any one of claims 21 to 32.

**38.** The use as claimed in claim 36 or claim 37 wherein said composition is at a pH of from 1-7 during exposure to said electrochemical energy.

**39.** The use as claimed in claim 38 wherein said pH is 2-6.

**40.** The use as claimed in any one of claims 36 to 39, wherein step (iii) comprises detecting and measuring the emitted radiation.

**41.** The use as claimed in any one of claims 36 to 40 wherein the method is a method of detecting or quantitating an analyte of interest by ECL competitive specific binding assay and wherein said composition further includes

(A) a sample to be tested for the analyte of interest,
(B)

(i) additional analyte of interest or an analog of the analyte of interest,
(ii) a reactive component capable of binding with (i),

and wherein the metal-containing ECL moiety is a metal chelate which is capable of entering into a binding interaction with the analyte of interest or with (B)(i) or (B)(ii).

**42.** The use as claimed in any one of claims 36 to 40 wherein the method is a method of detecting or quantitating an analyte of interest by ECL sandwich specific binding assay and wherein said composition further includes

(A) a sample to be tested for the analyte of interest,
(B)

(i) a binding partner of the analyte of interest, and
(ii) a reactive component capable of binding with (i),

and wherein the metal-containing ECL moiety is a metal chelate which is capable of entering into a binding interaction with the analyte of interest or with (B)(i) or (B)(ii).

**43.** The use as claimed in claim 41 or claim 42 in which the method is adapted for detection of an analyte selected from a whole cell or surface antigen, subcellular particle, virus, prion, viroid, antibody, antigen, hapten, fatty acid, nucleic acid, protein, lipoprotein, polysaccharide, lipopolysaccharide, glycoprotein, peptide, polypeptide, cellular metabolite, hormone, pharmacological agent, nonbiological polymer (preferably soluble), synthetic organic molecule, organometallic molecule, tranquillizer, barbiturate, alkaloid, steroid, vitamin, amino acid, sugar, lectin, recombinant or derived protein, biotin, avidin, streptavidin, or inorganic molecule present in the sample.

**44.** The use as claimed in claim 41, claim 42 or claim 43 wherein the composition is formed from a kit as claimed in any one of claims 33 to 35.

**45.** The use of a compound or compounds of the formula

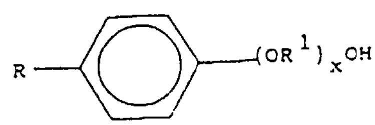

where R is hydrogen or $C_nH_{2n+1}$, $R^1$ is $C_nH_{2n}$, x is 0 to 70 and n is from 1 to 20, as an electromagnetic radiation enhancer in a system for detecting or quantitating an analyte of interest in a sample by competitive specific binding based upon an electrochemiluminescent phenomenon comprising:

(A) a sample;
(B)

(i) added analyte of interest or an analog of the analyte of interest,
(ii) a reactive component capable of binding with (i),
wherein one of said substances is linked directly or by one or more other molecules to a metal-containing ECL moiety which is capable of being converted to an excited state from which electromagnetic radiation is emitted upon exposure of the ECL moiety to conditions sufficient to induce said emission;

(C) a species which is capable of being converted to a strong reducing agent and an electrolyte;
(D) means for inducing the ECL moiety to emit electromagnetic radiation; and
(E) means for measuring the radiation emitted by said composition to determine the presence or quantity of the analyte of interest in the sample.

**46.** The use of a compound or compounds of formula

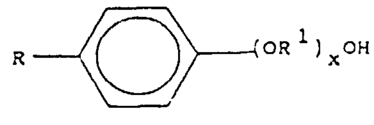

where R is hydrogen or $C_nH_{2n+1}$, $R^1$ is $C_nH_{2n}$, x is 0 to 70 and n is from 1 to 20, as an electromagnetic radiation enhancer in a system for detecting or quantitating an analyte of interest in a sample by sandwich specific binding based upon an electrochemiluminescent phenomenon comprising:

(A) a sample;

(B)

(i) a binding partner of the analyte of interest, and
(ii) a reactive component capable of binding with (i),
wherein one of said substances is linked directly or by one or more other molecules to a metal-containing ECL moiety which is capable of being converted to an excited state from which electromagnetic radiation is emitted upon exposure of the ECL moiety to conditions sufficient to induce said emission;

(C) a species which is capable of being converted to a strong reducing agent and an electrolyte;
(D) means for inducing the ECL moiety to emit electromagnetic radiation; and
(E) means for measuring the radiation emitted by said composition to determine the presence or quantity of the analyte of interest in the sample.

**47.** The use as claimed in claim 45 or claim 46 wherein the metal-containing ECL moiety is as specified in anyone of claims 2 to 4.

**48.** The use as claimed in claim 45, claim 46 or claim 47 wherein the species (c) is as specified in claim 5 or claim 6.

**49.** The use as claimed in any one of claims 45 to 48 wherein the electrolyte is as specified in any one of claims 9 to 17.

**50.** The use as claimed in any one of claims 45 to 49 wherein the compound of the formula

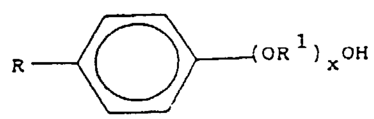

is as specified in claim 18 or claim 19.

**Patentansprüche**

**1.** Verwendung einer Verbindung oder von Verbindungen der Formel

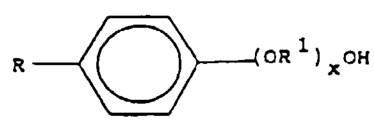

in der R Wasserstoff oder $C_{nH2+1}$ und R1 $C_nH_{2n}$ bedeutet, x 0 bis 70 und n 1 bis 20 ist, als Verstärker elektromagnetischer Strahlung in einer für die Verwendung in einem ECL-Test geeigneten Zubereitung, die enthält:

(a) eine metallhaltige ECL-Einheit, die, wenn durch Exposition an eine wirksame Menge elektrochemischer Energie oxidiert, in einen angeregten Zustand überführt werden kann, aus dem bei Exposition der angeregten ECL-Einheit an Emission induzierende Bedingungen elektromagnetische Strahlung emittiert wird,
(b) eine Spezies, die im oxidierten Zustand ein starkes Reduktionsmittel darstellt und
(c) einen Elektrolyten, der als Medium fungieren kann, in dem die ECL-Einheit und besagte Spezies oxidiert werden können.

**2.** Verwendung gemäß Anspruch 1, bei der die ECL-Einheit ein Metallchelat ist.

**3.** Verwendung gemäß Anspruch 2, bei der das Metall des Metallchelats ein Übergangsmetall oder ein Seltenerdmetall darstellt.

**4.** Verwendung gemäß Anspruch 3, bei der das Metall des Metallchelats Ruthenium, Osmium, Rhenium, Iridium, Rhodium, Platin, Indium, Palladium, Molybdän, Technetium, Kupfer, Chrom oder Wolfram ist.

5. Verwendung gemäß einem der Ansprüche 1 bis 4, wobei die Komponente (b) eine Carbonsäure ist.

6. Verwendung gemäß Anspruch 5, wobei die Carbonsäure Oxal-, Wein-, Zitronen-, Milch, Malon-, Glucon- oder Brenztraubensäure ist.

7. Verwendung gemäß einem der Ansprüche 1 bis 6, wobei die ein Reduktionsmittel bildende Spezies bezogen auf die ECL-Einheit in stöchiometrischem Überschuß vorliegt.

8. Verwendung gemäß Anspruch 5, Anspruch 6 oder Anspruch 7, bei der die Carbonsäure in einer Konzentration von 5 - 50 mM vorliegt.

9. Verwendung gemäß einem der vorstehenden Ansprüche, bei der der Elektrolyt ein wäßriger Elektrolyt ist.

10. Verwendung gemäß Anspruch 9, die einen Elektrolyten einschließt, der ein in Wasser gelöstes Salz umfaßt.

11. Verwendung gemäß Anspruch 9 oder 10, die einen Wasser und eine damit mischbare organische Flüssigkeit umfassenden Elektrolyten einschließt.

12. Verwendung gemäß einem der Ansprüche 9 bis 11, wobei der pH 1 bis 7 beträgt.

13. Verwendung gemäß Anspruch 12, wobei der pH 2 bis 6 beträgt.

14. Verwendung gemäß einem der Ansprüche 1 bis 8, bei der der Elektrolyt nicht wäßrig ist.

15. Verwendung gemäß einem der Ansprüche 1 bis 14, die einen eine organische Flüssigkeit umfassenden Elektrolyten einschließt.

16. Verwendung gemäß Anspruch 15, die einen Elektrolyten involviert, der Acetonitril, DMSO, DMF, Methanol, Ethanol oder eine Mischung aus zwei oder mehr derselben umfaßt.

17. Verwendung gemäß einem der Ansprüche 1 bis 16, die einen einen Phosphatpuffer umfassenden Elektrolyten involviert.

18. Verwendung gemäß einem der Ansprüche 1 bis 16, bei der in der Formel

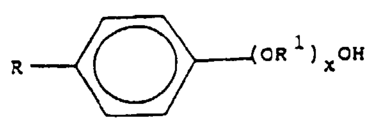

x 9 bis 10 ist.

19. Verwendung gemäß Anspruch 4, bei der das Metallchelat ein rutheniumhaltiges Chelat darstellt, die ein starkes Reduktionsmittel bildende Spezies Oxalat ist, der Elektrolyt einen Phosphatpuffer umfaßt und die Verbindung der Formel

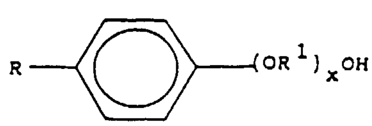

Triton-X-100® ist.

20. Verwendung gemäß einem der vorstehenden Ansprüche, bei der die Verbindung der Formel

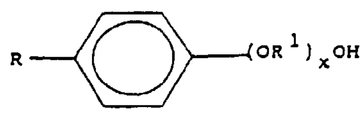

in einer Menge von 0,1 bis 1,0 Gew.-%/Vol. eingesetzt wird.

**21.** Verwendung gemäß einem der vorstehenden Ansprüche, bei der die Zubereitung in Form eines Kits vorliegt, wobei der Kit mindestens eine separate Komponente enthält, die eine oder mehrere Elemente der aus den Komponenten (a), (b) und (c) sowie die besagte Verbindung bestehenden Gruppe einschließt.

**22.** Verwendung gemäß Anspruch 21, bei der der Kit eine erste separate Komponente, die zwei beliebige Elemente der Gruppe einschließt, und eine zweite separate Komponente aufweist, die die restlichen Elemente der Gruppe einschließt.

**23.** Verwendung gemäß Anspruch 22, bei der die erste separate Komponente die ECL-Einheit (a) und den Elektrolyten (c) und die zweite separate Komponente die Spezies (b) und die besagte Ver- bindung enthält.

**24.** Verwendung gemäß Anspruch 21, bei der der Kit eine erste separate Komponente, die zwei beliebige Elemente der Gruppe ein- schließt, und eine zweite separate Komponente aufweist, die die restlichen Elemente der Gruppe sowie außerdem eines der in der ersten Komponente enthaltenen Elemente der Gruppe einschließt.

**25.** Verwendung gemäß Anspruch 24, bei der die erste separate Komponente die Spezies (b) und den Elektrolyten (c) und die zweite separate Komponente die ECL-Einheit (a) und die besagte Verbindung enthält.

**26.** Verwendung gemäß Anspruch 21, die vier separate Komponenten aufweist, wobei jede derselben ein anderes der vier Elemente der Gruppe enthält.

**27.** Verwendung gemäß Anspruch 21, die eine erste separate Komponente, die alle vier Elemente der Gruppe einschließt, und eine zweite separate Komponente aufweist, die ein, zwei oder drei beliebige Elemente der Gruppe umfaßt.

**28.** Verwendung gemäß Anspruch 27, bei der die zweite separate Komponente eines der vier Elemente der Gruppe enthält und der Kit darüber hinaus eine dritte separate Komponente, die eines, zwei oder drei Elemente der Gruppe umfaßt, und eine vierte separate Komponente aufweist, die ein oder zwei Elemente der Gruppe einschließt.

**29.** Verwendung gemäß Anspruch 22, bei der die erste separate Komponente die ECL-Einheit (a) und die Spezies (b) und die zweite separate Komponente den Elektrolyten (c) und die besagte Verbindung umfaßt.

**30.** Verwendung gemäß Anspruch 22, bei der die erste separate Komponente die ECL-Einheit (a) und die zweite separate Komponente die Spezies (b), den Elektrolyten (c) und die besagte Verbindung umfaßt.

**31.** Verwendung gemäß Anspruch 21, bei der der Kit eine erste separate Komponente, die mindestens zwei Elemente der Gruppe einschließt, und eine zweite separate Komponente aufweist, die das/die restlichen Elemente der Gruppe einschließt.

**32.** Verwendung gemäß einem der Ansprüche 22 bis 31, bei der jeweils die zweite Komponente wäßrig ist.

**33.** Verwendung gemäß einem der Ansprüche 21 bis 32, bei der der Kit an den Nachweis oder die Quantifizierung eines interessierenden Analyten mittels ECL angepaßt ist, wobei der Kit zusätzlich mindestens eine weitere Komponente aufweist, die ausgewählt ist unter (i) einem weiteren interessierenden Analyten oder einem Analogon des interessierenden Analyten, (ii) einem Bindungspartner des interessierenden Analyten oder seines Analogons und (iii) einer reaktiven Komponente, die die Substanz (i) oder (ii) binden kann, wobei die zusätzliche Substanz in einer weiteren, zusätzlich im Kit enthaltenen Komponente oder in einer Komponente des Kits enthalten ist, die eines oder mehr der Elemente der Gruppe enthält.

**34.** Verwendung gemäß Anspruch 33, bei der der Kit an den Nachweis eines Analyten adaptiert ist, der ausgewählt

ist unter einer ganzen Zelle oder einem Oberflächeantigen, subzellulären Partikeln, einem Virus, einem Prion, einem Viriod, einem Antikörper, einem Antigen, einem Hapten, einer Fettsäure, einer Nukleinsäure, einem Protein, einem Lipoprotein, einem Polysaccharid, einem Lipopolysaccharid, einem Glykoprotein, einem Peptid, einem Polypeptid, einem Zellmetaboliten, einem Hormon, einem pharmakologischen Wirkstoff, einem abiologischen Polymeren, einem synthetischen organischen Molekül, einem organometallischen Molekül, einem Tranquilizer, Barbiturat, Alkaloid, Steroid, Vitamin, einer Aminosäure, einem Zucker, Lectin, einem rekombinanten Protein oder Proteinderivat, Biotin, Avidin, Streptavidin oder einem anorganischen Molekül.

**35.** Verwendung gemäß Anspruch 33 oder 34, bei der der Kit an den Nachweis einer Probe des Analyten in einer Konzentration von 10-3 bis 10-18 Mol adaptiert ist.

**36.** Verwendung einer Verbindung oder von Verbindungen der Formel

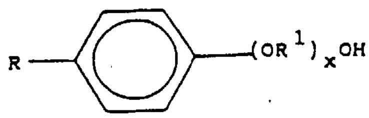

in der R Wasserstoff oder $C_{nH2n+1}$ und R1 $C_nH_{2n}$ bedeutet, x 0 bis 70 und n 1 bis 20 ist, als Verstärker elektromagnetischer Strah- lung in einem Verfahren zur Erzeugung einer Emission elektromagnetischer Strahlung, das die Schritte umfaßt:

(i) Herstellen einer Zubereitung gemäß einem der Ansprüche 1 bis 20,
(ii) Exponieren der Zubereitung unter geeigneten Bedingungen an eine Menge elektrochemischer Energie, die wirksam ist, um in der Zubereitung die Emission elektromagnetischer Energie zu induzieren, und
(iii) Nachweis der emittierten elektromagnetischen Strahlung,

wobei die Zubereitung die besagte Verbindung enthält.

**37.** Verwendung gemäß Anspruch 36, bei der die Zubereitung aus einem Kit gemäß einem der Ansprüche 21 bis 32 hergestellt wird.

**38.** Verwendung gemäß einem der Ansprüche 36 oder 37, bei der die Zubereitung während der Exposition an die elektrochemische Energie einen pH von 1 bis 7 aufweist.

**39.** Verwendung gemäß Anspruch 38, bei der der pH 2 - 6 beträgt.

**40.** Verwendung gemäß einem der Ansprüche 36 bis 39, bei der der Schritt (iii) den Nachweis und die Messung der emittierten Strahlung umfaßt.

**41.** Verwendung gemäß einem der Ansprüche 36 bis 40, bei der das Verfahren ein Verfahren zum Nachweis oder zur Quantifizierung eines interessierenden Analyten mittels eines ECL-Tests der kompetitiven spezifischen Bindung ist und bei der die Zubereitung zusätzlich umfaßt:

(A) eine auf den interessierenden Analyten zu testende Probe und
(B)

(i) einen weiteren interessierenden Analyten oder ein Analogon des interessierenden Analyten,
(ii) eine reaktive Komponente, die (i) binden kann,

und wobei die metallhaltige ECL-Einheit ein Metallchelat darstellt, das eine Bindungsreaktion mit dem interessierenden Analyten oder mit (B)(i) oder (B)(ii) eingehen kann.

**42.** Verwendung gemäß einem der Ansprüche 36 bis 40, bei der das Verfahren ein Verfahren zum Nachweis oder zur Quantifizierung eines interessierenden Analyten mittels eines ECL-Sandwichtests spezifischer Bindung ist und bei der die Zubereitung zusätzlich umfaßt:

(A) eine auf den interessierenden Analyten zu testende Probe und
(B)

(i) einen Bindungspartner des interessierenden Analyten,
(ii) eine reaktive Komponente, die (i) binden kann,

und wobei die metallhaltige ECL-Einheit ein Metallchelat darstellt, das eine Bindungsreaktion mit dem interessierenden Analyten oder mit (B)(i) oder (B)(ii) eingehen kann.

**43.** Verwendung gemäß Anspruch 41 oder 42, wobei das Verfahren an den Nachweis eines Analyten adaptiert ist, der ausgewählt ist unter einer ganzen Zelle oder einem Oberflächeantigen, subzellulären Partikeln, einem Virus, einem Prion, einem Viriod, einem Antikörper, einem Antigen, einem Hapten, einer Fettsäure, einer Nukleinsäure, einem Protein, einem Lipoprotein, einem Polysaccharid, einem Lipopolysaccharid, einem Glykoprotein, einem Pep- tid, einem Polypeptid, einem Zellmetaboliten, einem Hormon, einem pharmakologischen Wirkstoff, einem abiologischen Polymeren (vorzugsweise lösliche), einem synthetischen organischen Molekül, einem organometallischen Molekül, einem Tranquilizer, Barbiturat, Alkaloid, Steroid, Vitamin, einer Aminosäure, einem Zucker, Lectin, einem rekombinanten Protein oder Proteinderivat, Biotin, Avidin, Streptavidin oder einem anorganischen Molekül, die in der Probe vorliegen.

**44.** Verwendung gemäß Anspruch 41, 42 oder 43, bei der die Zubereitung aus eine Kit gemäß enem der Ansprüche 33 bis 35 hergestellt wird.

**45.** Verwendung einer Verbindung oder von Verbindungen der Formel

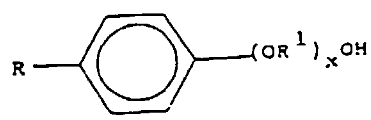

in der R Wasserstoff oder $C_{nH2n+1}$ und R1 $C_nH_{2n}$ bedeutet, x 0 bis 70 und n 1 bis 20 ist, als Verstärker elektromagnetischer Strahlung in einem System zum Nachweis oder zur Quantifizierung eines interessierenden Analyten in einer Probe mittels kompetitiver spezifischer Bindung basierend auf elektrochemischer Lumineszenz, umfassend:

(A) eine Probe,
(B)

(i) zugesetzten interessierenden Analyten oder ein Analogon des interessierenden Analyten,
(ii) eine zur Bindung an (i) befähigte reaktive Komponente,

wobei jede der Substanzen direkt oder über ein oder mehrere andere Moleküle an eine metallhaltige ECL-Einheit gebunden ist, die in eine angeregten Zustand überführt werden kann, aus dem bei Exposition der ECL-Einheit an Bedingungen, welche zur Induktion der Emission ausreichend sind, elektrochemische Strahlung emittiert wird,
(C) eine Spezies, die ind ein starkes Reduktionsmittel umgewandelt werden kann, und einen Elektrolyten,
(D) Mittel zur Induktion der Emission elektromagnetischer Strahlung bei der ECL-Einheit und
(E) Mittel zum Messen der von der Zubereitung emittierten Strahlung, um die Anwesenheit oder die Menge des Analyten in der Probe zu bestimmen.

**46.** Verwendung einer Verbindung oder von Verbindungen der Formel

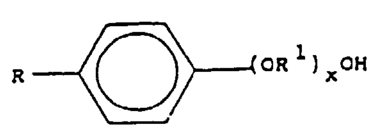

in der R Wasserstoff oder $C_{nH2n+1}$ und R1 $C_nH_{2n}$ bedeutet, x 0 bis 70 und n 1 bis 20 ist, als Verstärker elektromagnetischer Strahlung in einem System zum Nachweis oder zur Quantifizierung eines interessierenden Analyten in einer Probe mittels Sandwichspezifischer Bindung basierend auf elektrochemischer Lumineszenz, umfassend:

(A) eine Probe,
(B)

(i) einen Bindungspartner für den interessierenden Analyten,
(ii) eine zur Bindung an (i) befähigte reaktive Komponente,

wobei eine der Substanzen direkt oder über ein oder mehrere andere Moleküle an eine metallhaltige ECL-Einheit gebunden ist, die in eine angeregten Zustand überführt werden kann, aus dem bei Exposition der ECL-Einheit an Bedingungen, welche zur Induktion der Emission ausreichend sind, elektrochemische Strahlung emittiert wird,
(C) eine Spezies, die in ein starkes Reduktionsmittel umgewandelt werden kann, und einen Elektrolyten,
(D) Mittel zur Induktion der Emission elektromagnetischer Strahlung bei der ECL-Einheit und
(E) Mittel zum Messen der von der Zubereitung emittierten Strahlung, um die Anwesenheit oder die Menge des Analyten in der Probe zu bestimmen.

**47.** Verwendung gemäß Anspruch 45 oder 46, bei der die metallhaltige ECL-Einheit gemäß einem der Ansprüche 2 bis 4 ist.

**48.** Verwendung gemäß Anspruch 45, 46 oder 47, bei der die Spezies (c) gemäß einem der Ansprüche 5 oder 6 ist.

**49.** Verwendung gemäß einem der Ansprüche 45 bis 48, bei der der Elektrolyt gemäß einem der Ansprüche 9 bis 17 ist.

**50.** Verwendung gemäß einem der Ansrpüche 45 bis 49, bei die Verbindung der Formel

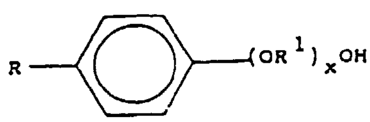

einem der Ansprüche 18 bis 19 entspricht.

## Revendications

**1.** Utilisation d'un composé, ou de composés de formule :

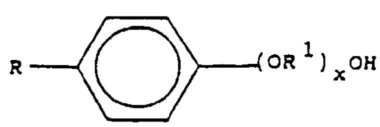

dans laquelle R représente un atome d'hydrogène, ou $C_nH_{2n+1}$, $R^1$ représente $C_nH_{2n}$, et n varie de 1 à 20, en tant que stimulateur de radiation électromagnétique dans une composition en vue de l'utilisation dans un dosage ECL, et comprenant :

- (a) une entité ECL contenant un métal, qui, lorsqu'elle est oxydée par exposition à une quantité efficace d'énergie électrochimique, est capable d'être portée à un état excité, à partir duquel une radiation électromagnétique est émise par exposition de l'entité ECL excitée, à des conditions suffisantes pour induire ladite émission ;
- (b) une espèce, qui lorsqu'elle est oxydée, forme un agent réducteur fort ;
- (c) un électrolyte capable de fonctionner comme un milieu dans lequel ladite entité ECL et ladite espèce

peuvent être oxydées.

2. Utilisation selon la revendication 1, dans laquelle l'entité ECL est un chélate métallique.

3. Utilisation selon la revendication 2, dans laquelle le métal dudit chélate métallique est un métal de transition ou un métal des terres rares.

4. Utilisation selon la revendication 3, dans laquelle le métal dudit chélate métallique est le ruthénium, l'osmium, le rhénium, l'iridium, le rhodium, le platine, l'indium, le palladium, le molybdène, le technétium, le cuivre, le chrome, ou le tungstène.

5. Utilisation selon l'une quelconque des revendications 1 à 4, dans laquelle le composant (b) est un acide carboxylique.

6. Utilisation selon la revendication 5, dans laquelle l'acide carboxylique est l'acide oxalique, l'acide tartrique, l'acide citrique, l'acide lactique, l'acide malonique, l'acide gluconique, ou l'acide pyruvique.

7. Utilisation selon l'une quelconque des revendications 1 à 6, dans laquelle ladite espèce formant un agent réducteur, est présente en excès stoechiométrique par rapport à l'entité ECL.

8. Utilisation selon la revendication 5, la revendication 6, ou la revendication 7, dans laquelle l'acide carboxylique est présent à une concentration comprise entre 5 et 50 mM.

9. Utilisation selon l'une quelconque des revendications précédentes, dans laquelle l'électrolyte est aqueux.

10. Utilisation selon la revendication 9, qui inclut un électrolyte comprenant un sel dissous dans l'eau.

11. Utilisation selon la revendication 9, ou la revendication 10, qui inclut un électrolyte comprenant l'eau, et un liquide organique miscible à l'eau.

12. Utilisation selon l'une quelconque des revendications 9 à 11, dans laquelle le pH est compris entre 1 et 7.

13. Utilisation selon la revendication 12, dans laquelle le pH est compris entre 2 et 6.

14. Utilisation selon l'une quelconque des revendications 1 à 8, dans laquelle ledit électrolyte est non aqueux.

15. Utilisation selon l'une quelconque des revendications 1 à 14, qui inclut un électrolyte comprenant un liquide organique.

16. Utilisation selon la revendication 15, qui inclut un électrolyte comprenant l'acétonitrile, le DMSO, le DMF, le méthanol, l'éthanol, ou un mélange de deux ou plusieurs de ceux-ci.

17. Utilisation selon l'une quelconque des revendications 1 à 16, qui inclut un électrolyte comprenant un tampon phosphate.

18. Utilisation selon l'une quelconque des revendications I à 16 dans laquelle, dans la formule :

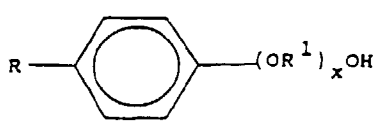

x est égale à 9 ou à 10.

19. Utilisation selon la revendication 4, dans laquelle le chélate métallique est un chélate contenant du ruthénium, l'espèce formant un agent réducteur fort est un oxalate, l'électrolyte comprend un tampon phosphate, et le composé de formule :

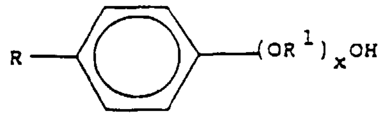

est le Triton X-100®.

**20.** Utilisation selon l'une quelconque des revendications précédentes, dans laquelle le composé de formule :

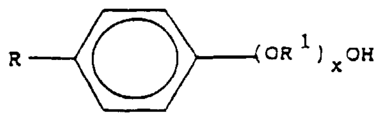

est incorporé en quantité comprise entre 0,1 et 1,0 pour cent exprimé en poids/volume.

**21.** Utilisation selon l'une quelconque des revendications précédentes, dans laquelle la composition est sous la forme d'un kit, ledit kit comprenant au moins un composant séparé incluant un ou plusieurs membres du groupe formé par les composants (a), (b), et (c), et ledit composé.

**22.** Utilisation selon la revendication 21, dans laquelle le kit consiste en un premier composant séparé incluant deux membres quelconques dudit groupe, et un second composant séparé incluant les membres restants dudit groupe.

**23.** Utilisation selon la revendication 22, dans laquelle le premier composant séparé contient ladite entité ECL (a), et ledit électrolyte (c), et ledit second composant séparé contient ladite espèce (b), et ledit composé.

**24.** Utilisation selon la revendication 21, dans laquelle le kit comprend un premier composant séparé qui inclut deux membres quelconques dudit groupe, et un second composant séparé incluant les membres restants dudit groupe, et également un des membres du groupe, qui est inclus dans ledit premier composant.

**25.** Utilisation selon la revendication 24, dans laquelle ledit premier composant séparé inclut ladite espèce (b), et ledit électrolyte (c), et ledit second composant séparé inclut ladite entité (a), et ledit composé.

**26.** Utilisation selon la revendication 21, qui comprend quatre composants séparés, chacun d'eux incluant un membre différent choisi parmi les quatre membres dudit groupe.

**27.** Utilisation selon la revendication 21, qui comprend un premier composant séparé incluant les quatre membres dudit groupe, et un second composant séparé incluant l'un quelconque, ou deux, ou trois des membres dudit groupe.

**28.** Utilisation selon la revendication 27, dans laquelle ledit second composant séparé inclut un des quatre membres dudit groupe, et ledit kit contient aussi un troisième composant séparé incluant un, deux, ou trois membres dudit groupe, et un quatrième composant séparé incluant un ou deux membres dudit groupe.

**29.** Utilisation selon la revendication 22, dans laquelle ledit premier composant séparé inclut ladite entité ECL (a), et ladite espèce (b), et ledit second composant séparé inclut ledit électrolyte (c), et ledit composé.

**30.** Utilisation selon la revendication 22, dans laquelle ledit premier composant séparé inclut ladite entité ECL (a), et ledit second composant séparé inclut ladite espèce (b), ledit électrolyte (c), et ledit composé.

**31.** Utilisation selon la revendication 21, dans laquelle le kit comprend un premier composant séparé incluant au moins deux membres dudit groupe, et un second composant séparé incluant le (ou les) membre(s) restant(s) dudit groupe.

**32.** Utilisation selon l'une quelconque des revendications 22 à 31, dans laquelle chaque second composant séparé est aqueux.

**33.** Utilisation selon l'une quelconque des revendications 21 à 32, dans laquelle le kit est adapté à détecter ou quantifier un analyte intéressant par dosage ECL, ledit kit comprenant également au moins une autre substance choisie parmi (i) un analyte intéressant additionnel, ou un analogue de l'analyte intéressant, (ii) un partenaire de liaison de l'analyte intéressant, ou dudit analogue de l'analyte intéressant, et (iii) un composant réactif capable de se lier avec la substance (i) ou (ii), ladite autre substance étant contenue dans un composant additionnel séparé inclus dans le kit, ou dans un composant dudit kit contenant un ou plusieurs des membres dudit groupe.

**34.** Utilisation selon la revendication 33, dans laquelle le kit est adapté à la détection d'un analyte choisi parmi un antigène de cellule entière ou de surface, une particule subcellulaire, un virus, un prion, un viroïde, un anticorps, un antigène, un haptène, un acide gras, un acide nucléique, une protéine, une lipoprotéine, un polysaccharide, un lipopolysaccharide, une glycoprotéine, un peptide, un polypeptide, un métabolite cellulaire, une hormone, un agent pharmacologique, un polymère non biologique, une molécule organique synthétique, une molécule organo-métallique, un tranquillisant, un barbiturate, un alcaloïde, un stéroïde, une vitamine, un acide aminé, un sucre, une lectine, une protéine recombinante ou dérivée, une biotine, une avidine, une streptavidine, ou une molécule minérale.

**35.** Utilisation selon la revendication 33 ou la revendication 34, dans laquelle le kit est adapté à la détection d'un spécimen d'analyte à une concentration molaire de $10^{-3}$ à $10^{-8}$.

**36.** Utilisation d'un composé, ou de composés de formule :

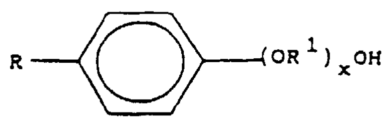

dans laquelle R représente un atome d'hydrogène, ou $C_nH_{2n+1}$, $R^1$ représente $C_nH_{2n}$, x varie de 0 à 70, et n varie de 1 à 20, en tant que stimulateur de radiation électromagnétique dans une méthode d'émission générant une radiation électromagnétique, qui comprend les étapes :

(i) de formation d'une composition selon l'une quelconque des revendications 1 à 20 :
(ii) d'exposition de la composition, dans des conditions convenables, à une quantité d'énergie électrochimique efficace pour induire la composition à émettre de l'énergie électromagnétique ;
(iii) de détection de la radiation électromagnétique émise ; ledit composé étant inclus dans ladite composition.

**37.** Utilisation selon la revendication 36, dans laquelle la composition est formée à partir d'un kit selon l'une quelconque des revendications 21 à 32.

**38.** Utilisation selon la revendication 36, ou la revendication 37, dans laquelle ladite composition est à un pH compris entre 1 et 7 au cours de l'exposition à ladite énergie électrochimique.

**39.** Utilisation selon la revendication 38, dans laquelle le pH est compris entre 2 et 6.

**40.** Utilisation selon l'une quelconque des revendications 36 à 39, dans laquelle l'étape (iii) comprend la détection et la mesure de la radiation émise.

**41.** Utilisation selon l'une quelconque des revendications 36 à 40, dans laquelle la méthode est une méthode de détection ou de quantification d'un analyte intéressant par dosage de liaison spécifique concurrente ECL, et dans laquelle ladite composition comprend aussi :

(A) un échantillon à tester pour l'analyte intéressant,

(B)(i) un analyte additionnel intéressant, ou un analogue de l'analyte intéressant,
(B)(ii) un composé réactif capable de se lier à (i),

et dans laquelle l'entité ECL contenant le métal est un chélate métallique qui est capable d'entrer en interaction de liaison avec l'analyte intéressant, ou avec (B)(i) ou (B)(ii).

**42.** Utilisation selon l'une quelconque des revendications 36 à 40, dans laquelle la méthode est une méthode de détection et de quantification d'un analyte intéressant par dosage de liaison spécifique sandwich ECL, et dans laquelle ladite composition comprend aussi :

(A) un échantillon à tester pour l'analyte intéressant,

(B)(i) un partenaire de liaison de l'analyte intéressant, et
(B)(ii) un composé réactif capable de se lier à (i),

et dans laquelle l'entité ECL contenant le métal est un chélate métallique qui est capable d'entrer en interaction de liaison avec l'analyte intéressant, ou avec (B)(i) ou (B)(ii).

**43.** Utilisation selon la revendication 41, ou la revendication 42, dans laquelle la méthode est adaptée à la détection d'un analyte choisi parmi un antigène de cellule entière ou de surface, une particule subcellulaire, un virus, un prion, un viroïde, un anticorps, un antigène, un haptène, un acide gras, un acide nucléique, une protéine, une lipoprotéine, un polysaccharide, un lipopolysaccharide, une glycoprotéine, un peptide, un polypeptide, un métabolite cellulaire, une hormone, un agent pharmacologique, un polymère non biologique (de préférence soluble), une molécule organique synthétique, une molécule organo-métallique, un tranquillisant, un barbiturate, un alcaloïde, un stéroïde, une vitamine, un acide aminé, un sucre, une lectine, une protéine recombinante ou dérivée, une biotine, une avidine, une streptavidine, ou une molécule minérale, présent dans l'échantillon.

**44.** Utilisation selon la revendication 41, le revendication 42, ou la revendication 43, dans laquelle la composition est formée à partir d'un kit selon l'une quelconque des revendications 33 à 35.

**45.** Utilisation d'un composé, ou de composés de formule :

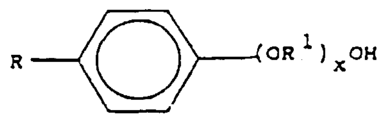

dans laquelle R représente un atome d'hydrogène, ou $C_nH_{2n+1}$, $R^1$ représente $C_nH_{2n}$, x varie de 0 à 70, et n varie de 1 à 20, en tant que stimulateur de radiation électromagnétique dans un système de détection ou de quantification d'un analyte intéressant dans un échantillon par liaison spécifique concurrente, basée sur un phénomène électroluminescent, comprenant :

(A) un échantillon ;
(B)

(i) un analyte intéressant ajouté ou un analogue de l'analyte intéressant
(ii) un composant réactif capable de se lier à (i), dans laquelle une desdites substances est liée directement, ou par une ou plusieurs autres molécules, à une entité ECL contenant un métal, qui est capable d'être portée à un état excité à partir duquel la radiation électromagnétique est émise lors de l'exposition de l'entité ECL à des conditions suffisantes pour induire ladite émission ;

(C) une espèce qui est capable d'être convertie en un agent réducteur fort, et un électrolyte ;
(D) des moyens induisant l'entité ECL à émettre la radiation électromagnétique ; et
(E) des moyens de mesure de la radiation électromagnétique émise par ladite composition pour déterminer la présence, ou la quantité de l'analyte intéressant dans l'échantillon.

**46.** Utilisation d'un composé, ou de composés de formule :

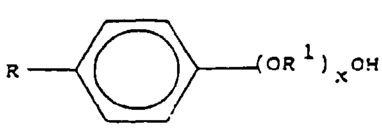

dans laquelle R représente un atome d'hydrogène, ou $C_nH_{2n+1}$, $R^1$ représente $C_nH_{2n}$, x varie de 0 à 70, et n varie de 1 à 20, en tant que stimulateur de radiation électromagnétique dans un système de détection ou de quantification d'un analyte intéressant dans un échantillon par liaison spécifique sandwich, basée sur un phénomène électroluminescent, comprenant :

(A) un échantillon ;
(B)

(i) un partenaire de liaison de l'analyte intéressant, et
(ii) un composant réactif capable de se lier à (i), dans laquelle une desdites substances est liée directement, ou par une ou plusieurs autres molécules, à une entité ECL contenant un métal, qui est capable d'être portée à un état excité à partir duquel la radiation électromagnétique est émise lors de l'exposition de l'entité ECL à des conditions suffisantes pour induire ladite émission ;

(C) une espèce qui est capable d'être convertie en un agent réducteur fort, et un électrolyte ;
(D) des moyens induisant l'entité ECL à émettre la radiation électromagnétique ; et
(E) des moyens de mesure de la radiation électromagnétique par ladite composition pour déterminer la présence, ou la quantité de l'analyte intéressant dans l'échantillon.

**47.** Utilisation selon la revendication 45, ou la revendication 46, dans laquelle l'entité ECL contenant un métal est telle que spécifiée dans l'une quelconque des revendications 2 à 4.

**48.** Utilisation selon la revendication 45, la revendication 46, ou le revendication 47, dans laquelle l'espèce (c) est telle que définie dans la revendication 5 ou la revendication 6.

**49.** Utilisation selon l'une quelconque des revendications 45 à 48, dans laquelle l'électrolyte est tel que spécifié dans l'une quelconque des revendications 9 à 17.

**50.** Utilisation selon l'une quelconque des revendications 45 à 49, dans laquelle la composé de formule :

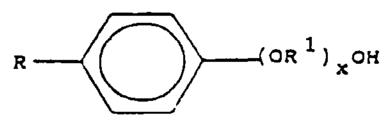

est tel que spécifié dans la revendication 18 ou la revendication 19.

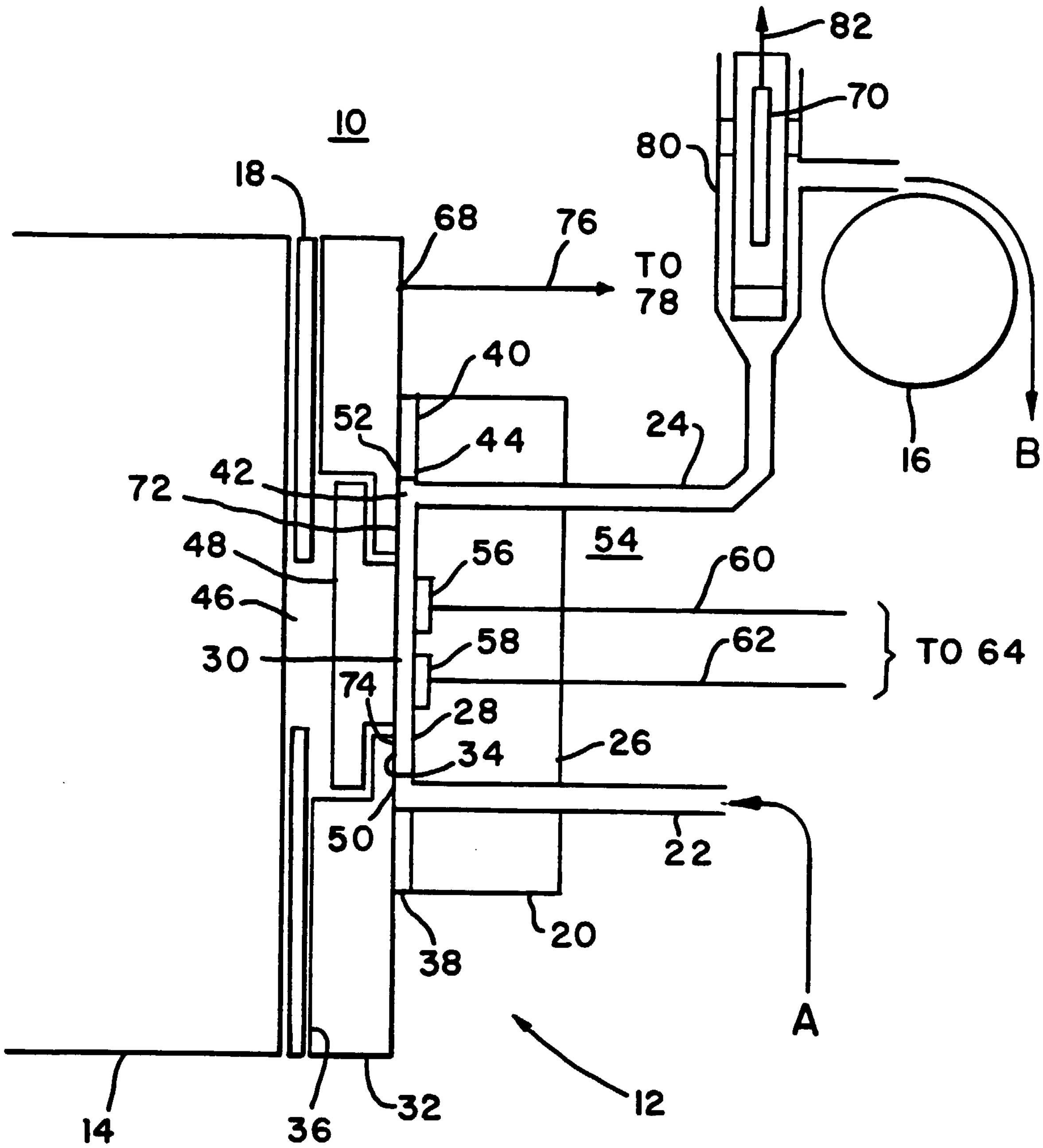
10
18
68
76
TO
78
82
70
80
40
52
44
24
16
B
42
72
48
54
56
60
46
58
62
TO 64
30
74
28
34
26
50
22
20
38
A
14
36
32
12

FIG. 1

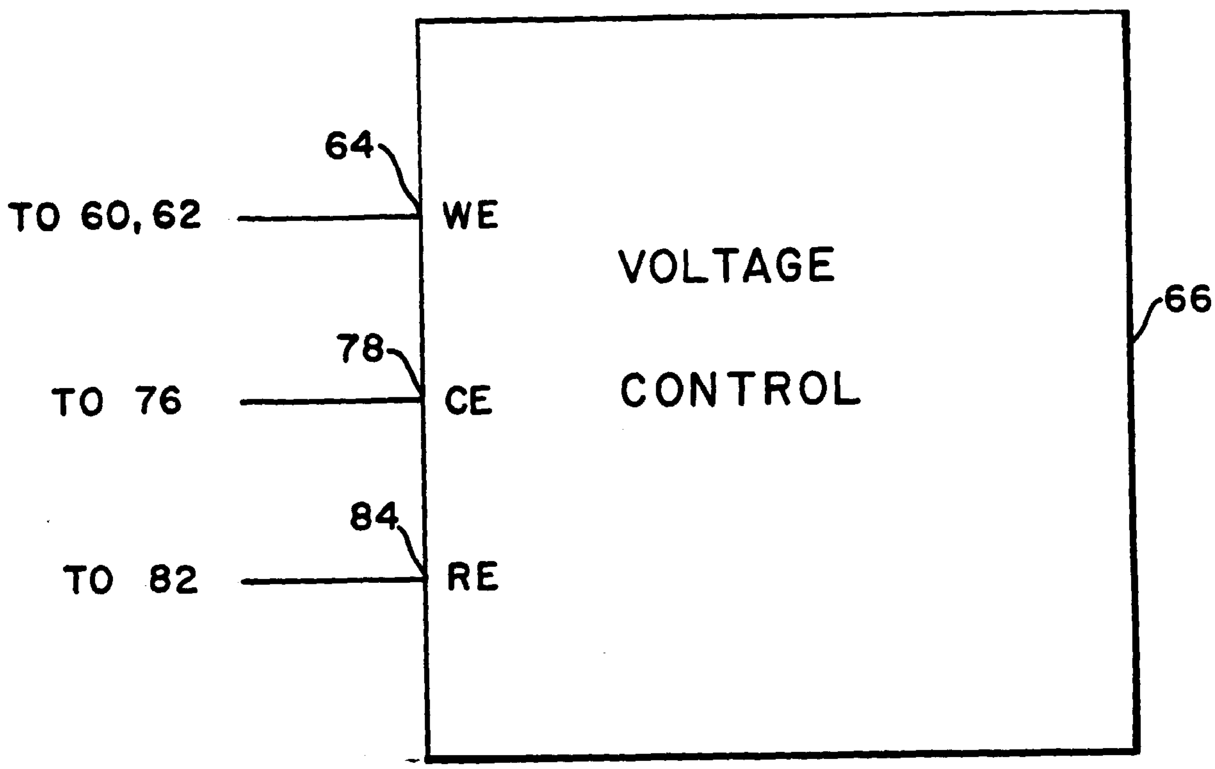


FIG. 2

Fig. 3

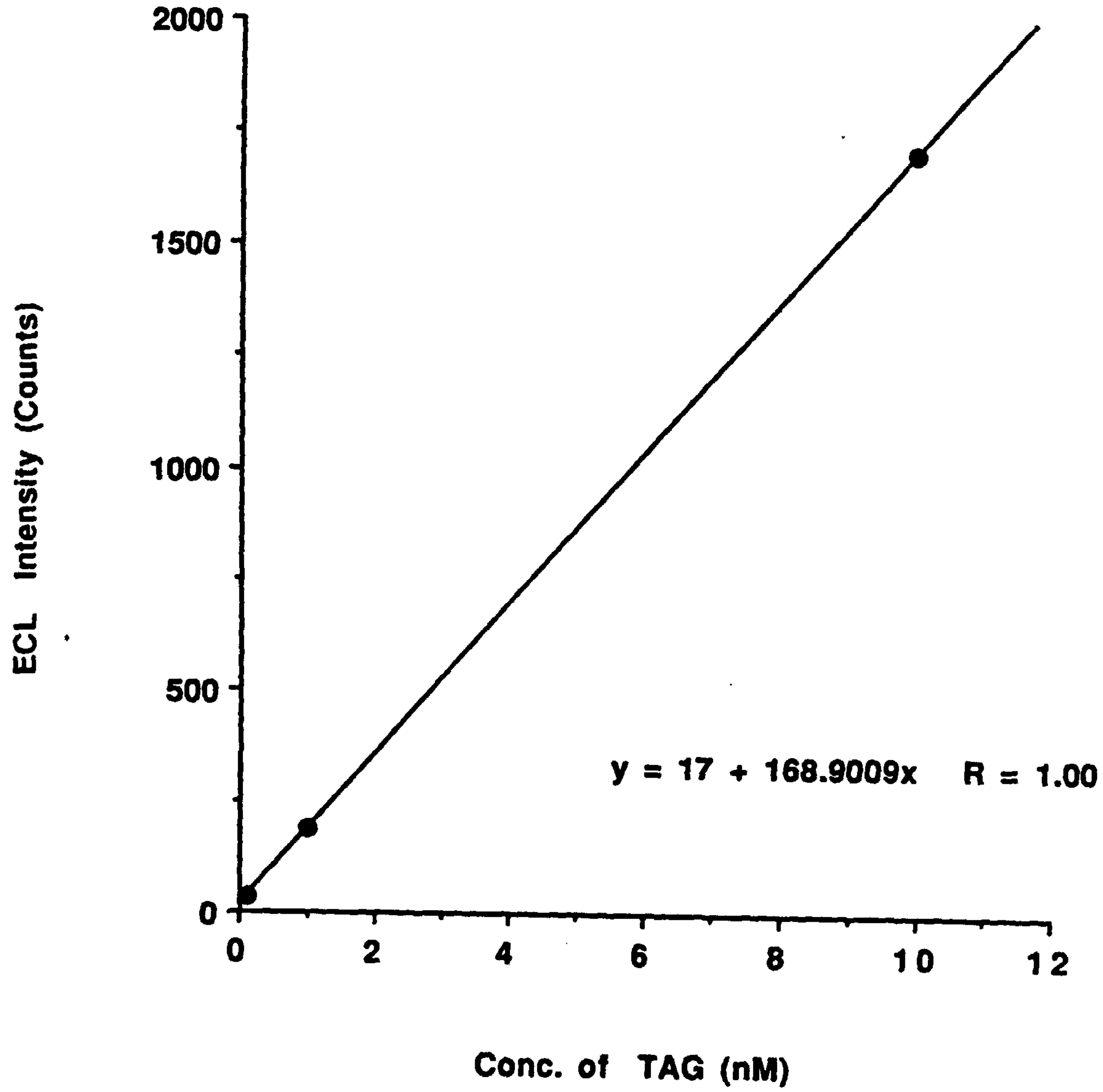
2000
1500
1000
500
0
ECL Intensity (Counts)
y = 17 + 168.9009x R = 1.00
0
2
4
6
8
10
12
Conc. of TAG (nM)

Fig. 4

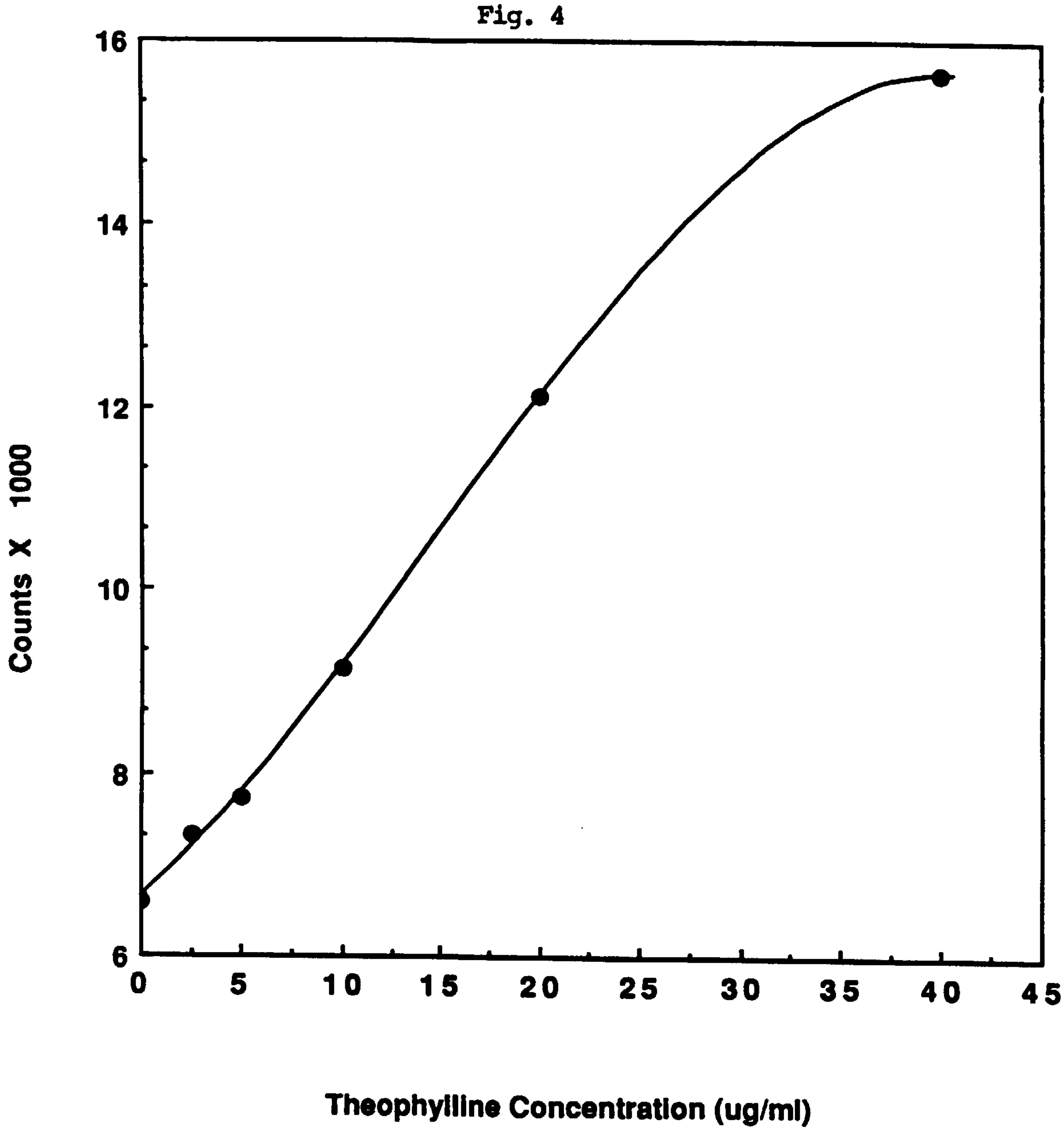
16
14
12
10
8
6
Counts X 1000
0
5
10
15
20
25
30
35
40
45
Theophylline Concentration (ug/ml)

Fig. 5

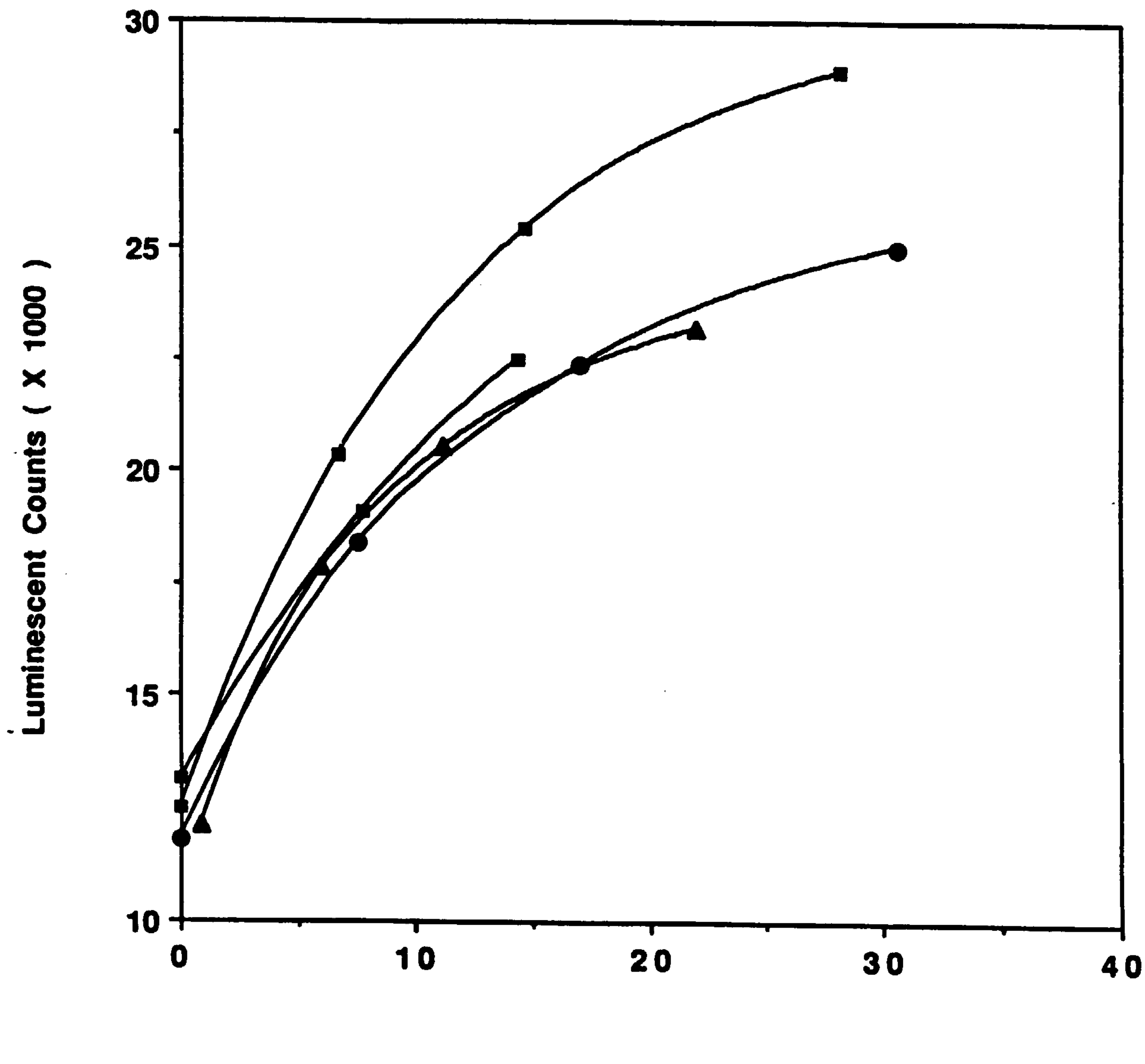
30
25
20
15
10
0
10
20
30
40
Luminescent Counts ( X 1000 )
Theophylline Concentration ( µg/ml )

Fig. 6 A

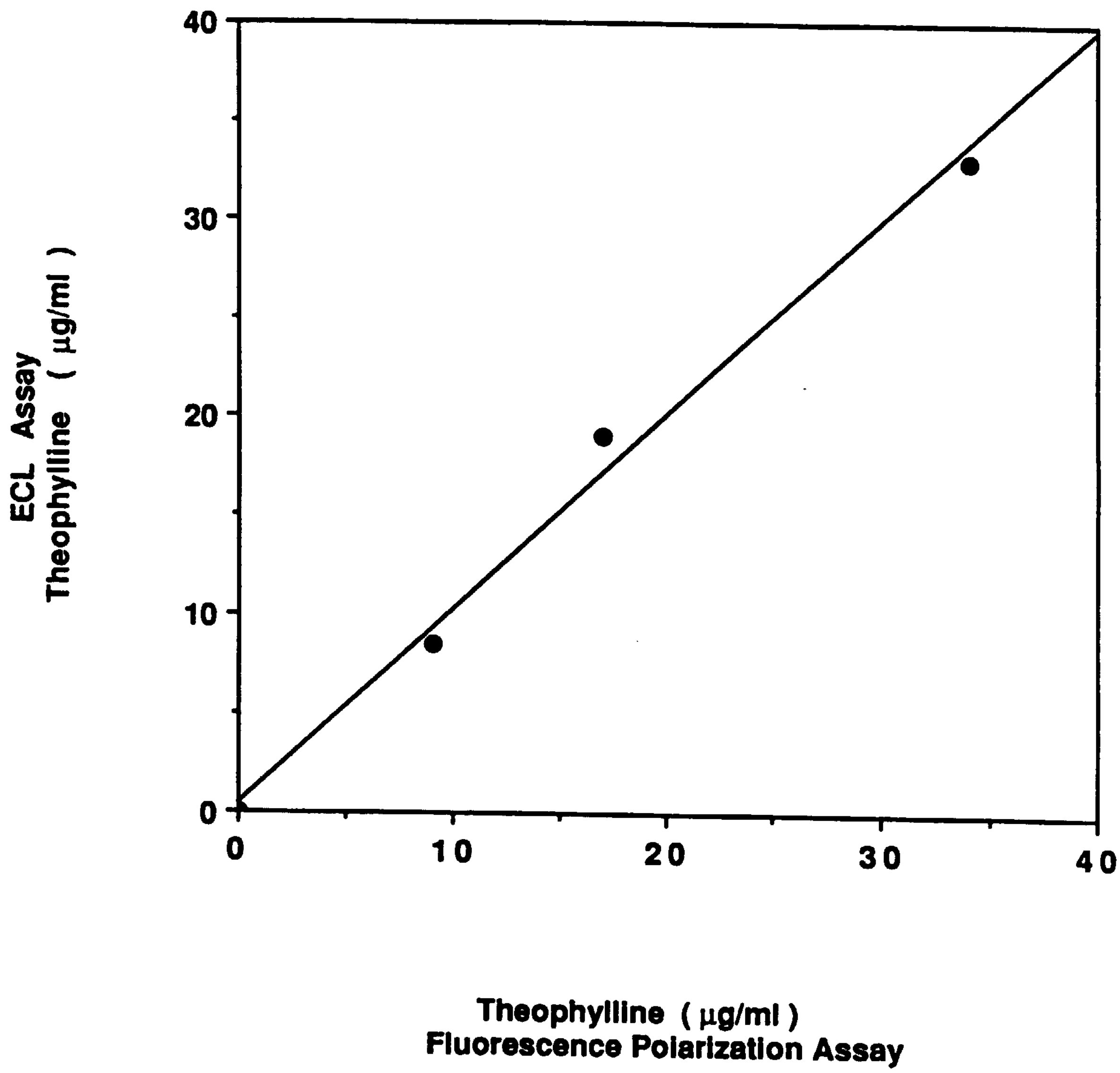
40
30
20
10
0
0
10
20
30
40
ECL Assay
Theophylline ( µg/ml )
Theophylline ( µg/ml )
Fluorescence Polarization Assay

Fig. 6B

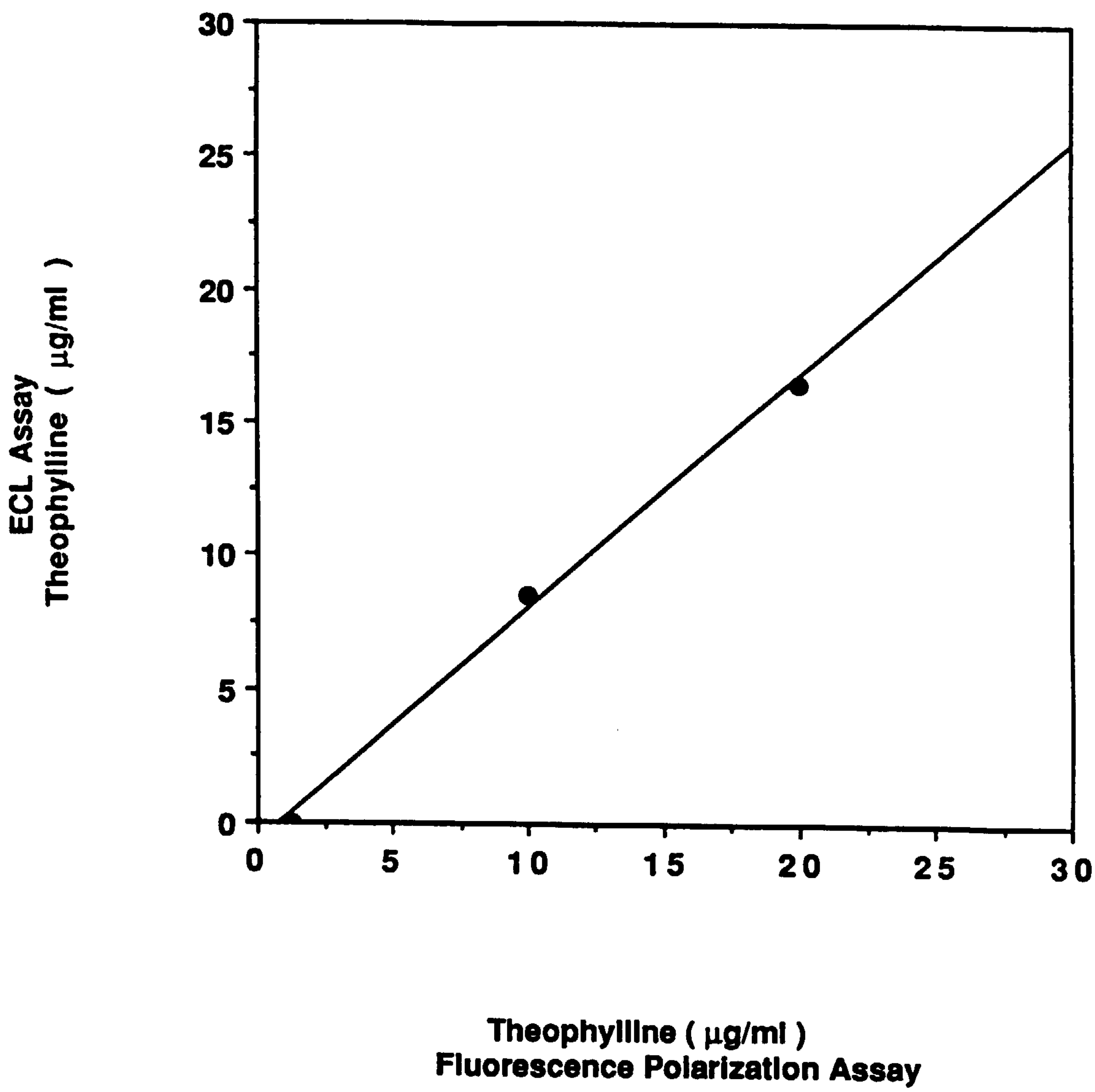
ECL Assay
Theophylline ( μg/ml )
30
25
20
15
10
5
0
0
5
10
15
20
25
30
Theophylline ( μg/ml )
Fluorescence Polarization Assay

Fig. 6 C

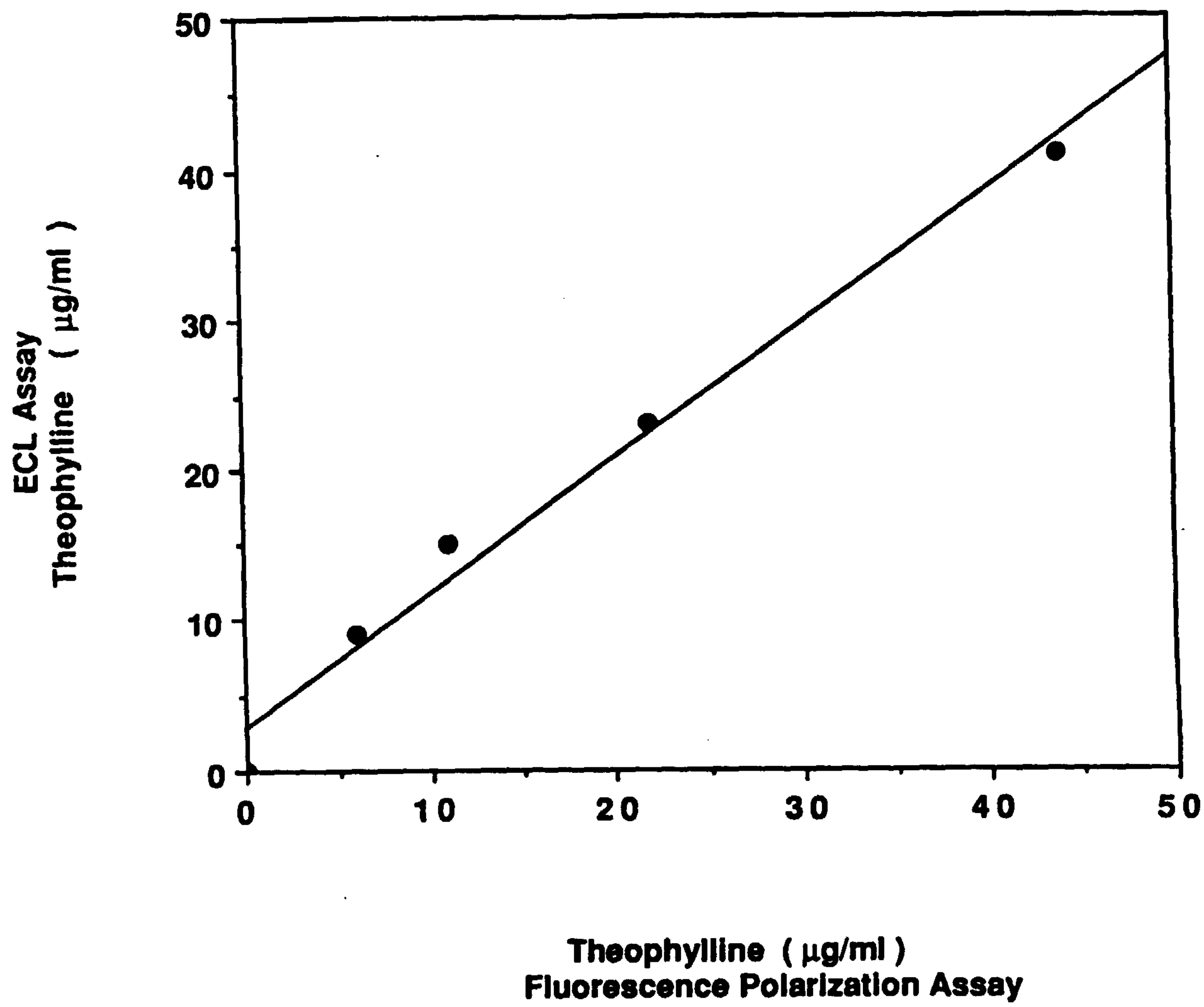
50
40
30
20
10
0
ECL Assay
Theophylline ( µg/ml )
0
10
20
30
40
50
Theophylline ( µg/ml )
Fluorescence Polarization Assay

Fig. 6 D

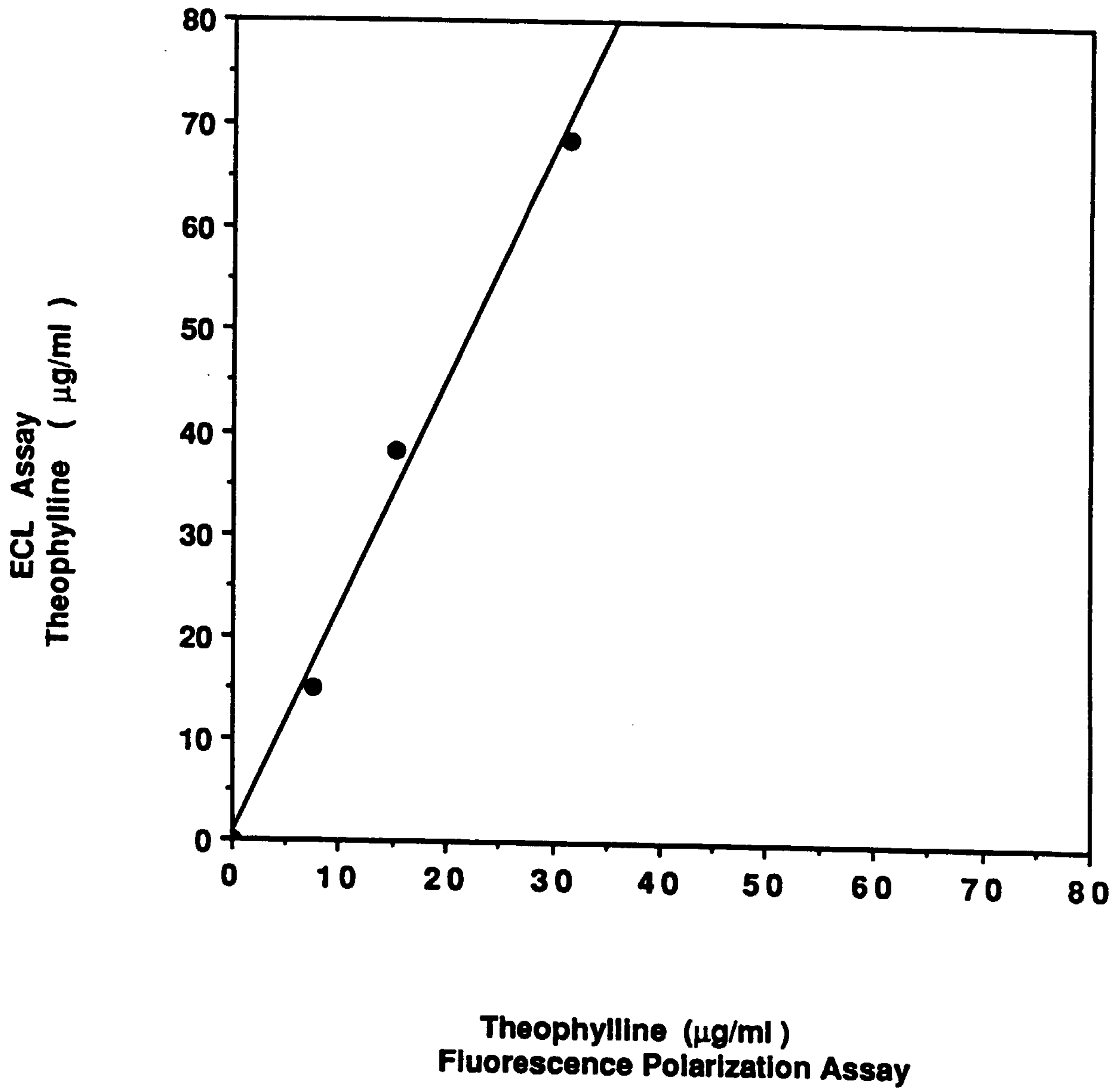
ECL Assay
Theophylline ( µg/ml )
0
10
20
30
40
50
60
70
80
0
10
20
30
40
50
60
70
80
Theophylline (µg/ml )
Fluorescence Polarization Assay

Fig. 7

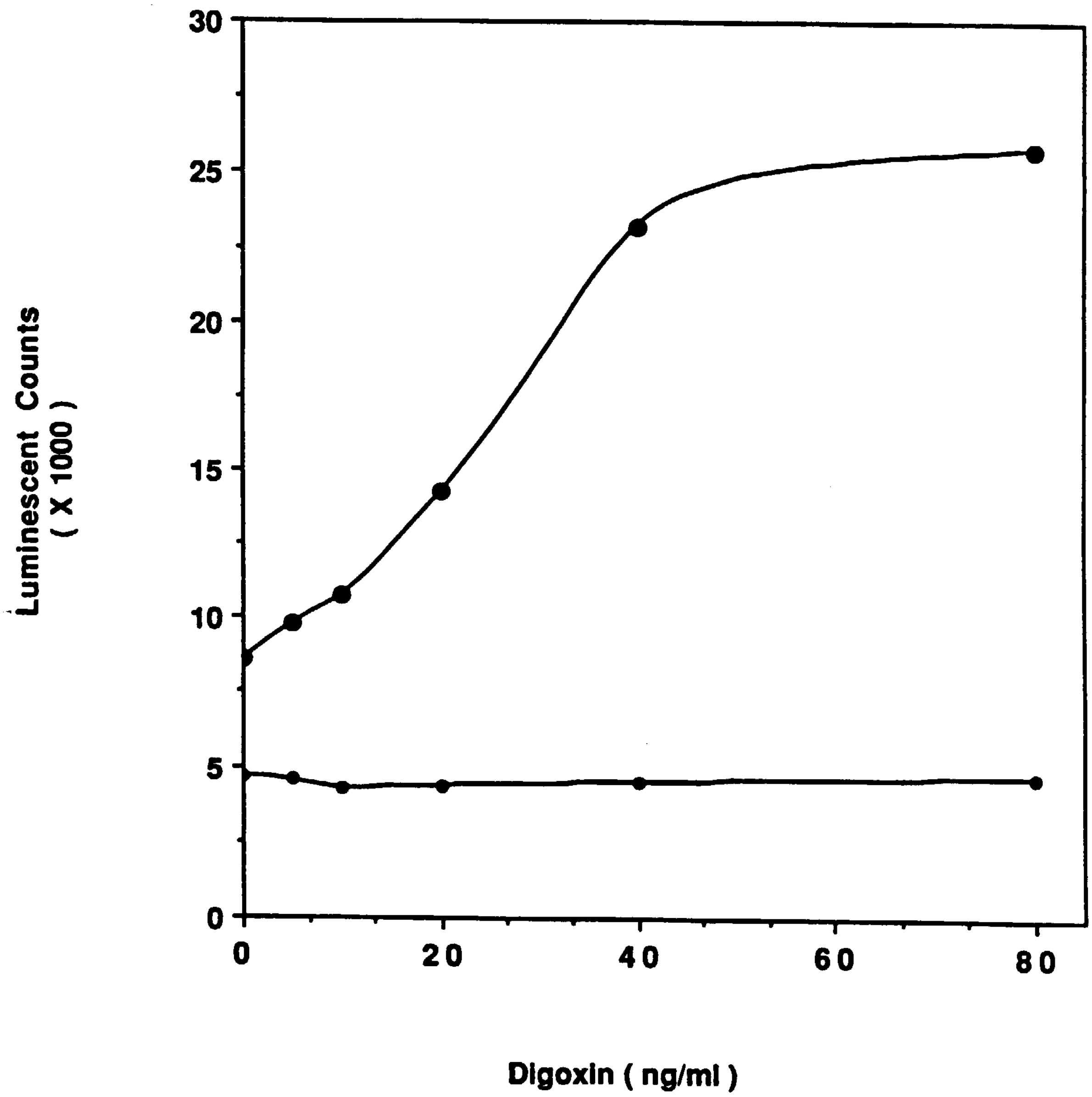
30
25
20
15
10
5
0
Luminescent Counts
( X 1000 )
0
20
40
60
80
Digoxin ( ng/ml )